# FASCICULE DE BREVET EUROPEEN

(11) **EP 1 532 965 B1**
(45) Date de publication et mention de la délivrance du brevet: **22.08.2007**
(21) Numéro de dépôt: 04292604.8
(22) Date de dépôt: 03.11.2004
(51) Int. Cl.: A61Q 5/06, A61K 8/81

(54) **Composition cosmétique à base d'un composé cosmétiquement actif et d'un gel comprenant au moins un réseau réticulé de particules de polymère réticulé**
Kosmetische Zusammensetzung auf der Basis einer kosmetisch wirksamen Verbindung und eines Gels, das mindestens ein vernetztes Netzwerk von Partikeln eines vernetzten Polymers enthält.
Cosmetic composition based on a cosmetical active ingredient and an gel comprising at least one reticulated network of reticulated polymer particles.

(30) Priorité: 18.11.2003 FR 0313448
(43) Date de publication de la demande: 25.05.2005
(73) Titulaire: L'ORÉAL, 75008 Paris (FR)
(72) Inventeur: Mathonneau, Estelle, 75010 Paris (FR); Rollat, Isabelle, 75017 Paris (FR); Giroud, Franck, 92100 Clichy (FR)
(74) Mandataire: Dossmann, Gérard

(56) Documents cités:
- WO-A-03/022910
- HU Z ET AL: "POLYMER GEL NANOPARTICLE NETWORKS" ADVANCED MATERIALS, VCH VERLAGSGESELLSCHAFT, WEINHEIM, DE, vol. 12, no. 16, 16 août 2000 (2000-08-16), pages 1173-1176, XP000963581 ISSN: 0935-9648

## Description

La présente invention est relative à de nouvelles compositions cosmétiques, et notamment capillaires, à base d'un composé cosmétiquement actif et d'un gel comprenant au moins un réseau réticulé de particules de polymère réticulé. Elle concerne également un procédé de traitement cosmétique des matières kératiniques, et plus particulièrement des fibres kératiniques telles que les cheveux, ainsi que l'utilisation en cosmétique de ces compositions. Par composé cosmétiquement actif, on entend un produit agréable à toucher, ayant un aspect esthétique et une odeur agréable, utilisable tous les jours, sans prescription médicale, pendant plus de six mois.

Les produits de coiffage permettant la formation de mèches sont généralement des gels contenant un polymère fixant. L'inconvénient lié à ce type de produit réside dans le fait que la tenue de ces mèches est limitée dans le temps et que le polymère fixant incorporé dans le gel donne aux mèches un toucher collant, très peu cosmétique.

Il est connu d'utiliser un gel comprenant au moins un réseau réticulé de particules de polymère réticulé, tel que décrit dans la demande de brevet WO 03/022910. Toutefois, les compositions à base de gel divulguées dans ce document comprennent un composé pharmaceutique et sont destinées à des applications médicales. Il n'est pas fait mention de l'association du gel avec un composé cosmétiquement actif.

On connaît par ailleurs des traitements permanents des fibres kératiniques, et en particulier des cheveux. Ces traitements utilisent un agent réducteur et un agent oxydant, et nécessitent une mise sous tension mécanique des cheveux à l'aide d'un matériel d'enroulement, afin de conférer une mise en forme durable de la chevelure. Bien qu'ils permettent de fixer la coiffure de manière plus durable que les gels associés à des polymères fixants, ces procédés présentent l'inconvénient de dégrader la qualité de toucher des cheveux.

La présente invention se propose de remédier à ces inconvénients.

En particulier, la Demanderesse vient de découvrir de façon surprenante que des compositions cosmétiques comprenant, dans un milieu cosmétiquement acceptable, un composé cosmétiquement actif et un gel comprenant au moins un réseau réticulé de particules de polymère réticulé conféraient aux cheveux une bonne fixation et des propriétés cosmétiques durables, et en particulier la formation de mèches rigides plus fines qu'avec les gels de coiffage de l'art antérieur, en particulier ceux contenant un polymère fixant.

Elle a en outre constaté que les compositions selon l'invention conféraient aux cheveux une protection de leur surface et un toucher agréable. En outre, ces compositions assurent un gainage du cheveu.

D'autres objets de l'invention apparaîtront à la lecture de la description et des exemples qui suivent.

La présente invention a donc pour objet une composition cosmétique, notamment capillaire, caractérisée en ce qu'elle est comprend, dans un milieu cosmétiquement acceptable, au moins un composé cosmétiquement actif et un gel comprenant au moins un réseau réticulé de particules de polymère réticulé.

L'invention a également pour objet l'utilisation d'un gel comprenant au moins un réseau réticulé de particules de polymère réticulé pour la fabrication d'une composition cosmétique, notamment capillaire, destinée en particulier au maintien et/ou à la mise en forme de la coiffure.

Un autre objet de l'invention concerne un procédé cosmétique capillaire, en particulier un procédé de fixation et/ou de maintien de la coiffure mettant en oeuvre les compositions selon l'invention.

Par composé cosmétiquement actif, on entend, au sens de la présente invention, tout composé actif ayant une activité cosmétique, non médicale. Le composé cosmétiquement actif au sens de l'invention est un produit qui peut être obtenu sans ordonnance ou prescription médicale pendant plus de six mois.

Le composé cosmétiquement actif peut être choisi parmi les agents réducteurs ou antioxydants, les agents oxydants, les silicones, les agents adoucissants, les agents anti-mousse, les agents hydratants, les agents émollients, les plastifiants, les filtres solaires, les colorants, les pigments, les charges, les argiles, les nacres ou agents nacrants, les parfums, les peptisants, les agents épaississants, les conservateurs, les polymères fixants, les vitamines ou provitamines, les agents conditionneurs tels que les huiles, les polymères cationiques, les tensioactifs cationiques, les cires dont les céramides, les protéines.

L'agent réducteur peut être choisi parmi les réducteurs organiques ou minéraux tels que NaBH₄, KBH₄, BH₃, NADPH, NADH, H₂, 9-BBN (9 borabicyclo[[3.3.1]nonane), LiAlH₄, NaAlEt₂H₂, Na₂S₂O₃, le diisoamylborane, l'acide ascorbique et les composés cycliques ou aliphatiques contenant le groupe chimique enediol(OH)C=C(OH)- incluant les formes isomériques, leurs sels et leurs esters, l'hydroquinone, NaBH(OAc)₃, l'acide formamidine sulfinique (FASA), l'acide N-phénylformadimine sulfinique, les phosphines, NaBH₃CN, Na₂S₂O₄, BMS (bismercaptoéthylsulfone), DTT (dithiothréitol), DMH (N,N'diméthyl-N,N'-bis(mercaptoacétyl)hydrazine), les thiols, les thiolates, les citrates, les sulfites, bisulfites, monopersulfates et bisulfates de métaux alcalins, les enzymes du type protéine disulfure isomérase ou la thiorédoxine, et les sulfinates, en particulier les hydroxyméthane sulfinates.

L'agent oxydant peut être choisi parmi l'eau oxygénée, les bromates et les persels, comme par exemple les persulfates, les percarbonates.

L'agent épaississant peut être choisis parmi les épaississants naturels tels que la gomme de guar, la gomme de xanthane et la farine d'épicéa, la cellulose, leurs dérivés modifiés comme l'hydroxyéthylcellulose, l' hydroxypropylguar, la carboxyéthylcellulose, et les polymères synthétiques de caractère associatif ou non tels que les polymères à base de monomères acryliques et les polycondensats du type polyuréthane. Ces agents épaississants peuvent être de nature non ionique, anionique, cationique ou amphotère.

Le filtre solaire peut être choisi parmi les anthranilates; les dérivés cinnamiques ; les dérivés de dibenzoylméthane; les dérivés salicyliques ;les dérivés du camphre ; les dérivés de triazine, de préférence les dérivés de 1,3,5 triazine, tels que ceux décrits dans les demandes de brevet US 4,367,390, US 4,724,137, EP 863 145, EP 517 104, EP 570 838, EP 796 851, EP 775 698, EP 878 469, EP 933 376, EP 507 691, EP 507 692 , EP 790 243 et EP 944 624 ; les dérivés de la benzophénone; les dérivés de β-β'-diphénylacrylate ; les dérivés de benzotriazole; les dérivés de benzalmalonate; les dérivés de benzimidazole; les imidazolines ; les dérivés bis-benzoazolyle tels que décrits dans les brevets EP 669 323 et US 2,463,264 ; les dérivés de l'acide p-aminobenzoïque (PABA) ; les dérivés de méthylène bis-(hydroxyphényl benzotriazole) tels que décrits dans les demandes US 5,237,071, US 5,166,355, GB 2303546, DE 19726184 et EP 893 119 ;les polymères filtres et silicones filtres tels que ceux décrits notamment dans la demande WO 93/04665 ; les dimères dérivés d'α-alkylstyrène tels que ceux décrits dans la demande de brevet DE 19855649 ; les 4,4-diarylbutadiènes tels que décrits dans les demandes EP 0 967 200 et DE 19755649 et leurs mélanges.

Parmi les dérivés du dibenzoylméthane, on préfère tout particulièrement mettre en oeuvre le 4-(tert.-butyl)4'-méthoxydibenzoylméthane, ou Butyl Methoxy Dibenzoylmethane, notamment vendu sous la dénomination commerciale de « Parsol® 1789 » par la société HOFFMANN LAROCHE.

Les colorants capillaires peuvent être des colorants directs ou des colorants d'oxydation.

Les colorants d'oxydation utilisables selon l'invention sont choisis parmi les bases d'oxydation et/ou les coupleurs.

De préférence les compositions selon l'invention contiennent au moins une base d'oxydation.

Les bases d'oxydation utilisables dans le cadre de la présente invention sont choisies parmi celles classiquement connues en teinture d'oxydation, et parmi lesquelles on peut notamment citer les ortho- et para-phénylènediamines, les bases doubles, les ortho- et para-aminophénols, les bases hétérocycliques, ainsi que leurs sels d'addition avec un acide.

Parmi les bases hétérocycliques utilisables à titre de bases d'oxydation dans les compositions conformes à l'invention, on peut plus particulièrement citer les dérivés pyridiniques, les dérivés pyrimidiniques, les dérivés pyrazoliques, et leurs sels d'addition avec un acide.

Les coupleurs utilisables comme colorant sont ceux classiquement utilisés dans les compositions de teinture d'oxydation, c'est-à-dire les méta-aminophénols, les méta-phénylènediamines, les métadiphénols, les naphtols et les coupleurs hétérocycliques tels que par exemple les dérivés indoliques, les dérivés indoliniques, le sésamol et ses dérivés, les dérivés pyridiniques, les dérivés pyrazolotriazoles, les pyrazolones, les indazoles, les benzimidazoles, les benzothiazoles, les benzoxazoles, les 1,3-benzodioxoles, les quinolines et leurs sels d'addition avec un acide.

D'une manière générale, les sels d'addition avec un acide des bases d'oxydation et coupleurs sont notamment choisis parmi les chlorhydrates, les bromhydrates, les sulfates et les tartrates, les lactates et les acétates.

Le colorant peut être choisi parmi les colorants directs, naturels ou synthétiques. Ces colorants directs peuvent notamment alors être choisis parmi les colorants nitrés, azoïques méthiniques ou quinoniques, triarylméthaniques, neutres, cationiques ou anioniques, les extraits de plantes.

Les pigments peuvent être blancs ou colorés, minéraux et/ou organiques, enrobés ou non. On peut citer, parmi les pigments minéraux, le dioxyde de titane, éventuellement traité en surface, les oxydes de zirconium, de zinc ou de cérium, ainsi que les oxydes de fer ou de chrome, le violet de manganèse, le bleu outremer, l'hydrate de chrome et le bleu ferrique. Parmi les pigments organiques, on peut citer le noir de carbone, les pigments de type D & C, et les laques à base de carmin de cochenille, de baryum, strontium, calcium, aluminium.

Les nacres ou agents nacrants peuvent être choisis parmi les pigments nacrés blancs tels que le mica recouvert de titane ou d'oxychlorure de bismuth, les pigments nacrés colorés tels que le mica titane avec des oxydes de fer, le mica titane avec seulement du bleu ferrique ou de l'oxyde de chrome, le mica titane avec notamment du bleu ferrique ou de l'oxyde de chrome, le mica titane avec un pigment organique du type précité ainsi que les pigments nacrés à base d'oxychlorure de bismuth, les éthers ou esters organiques insolubles.

Au sens de la présente invention, on entend par polymère fixant tout polymère, filmogène ou non, permettant de donner une forme à la chevelure ou de maintenir cette forme.

Les polymères fixants convenant dans l'invention sont notamment choisis parmi les polymères fixants cationiques, anioniques, amphotères, non ioniques et leurs mélanges.

Les polymères fixants cationiques utilisables selon la présente invention sont de préférence choisis parmi les polymères comportant des groupements amine primaire, secondaire, tertiaire et/ou quaternaire faisant partie de la chaîne polymère ou directement reliés à celle-ci, et ayant une masse moléculaire moyenne en nombre comprise entre 500 et environ 5 000 000, et de préférence entre 1 000 et 3 000 000.

Parmi ces polymères, on peut citer plus particulièrement les polymères cationiques suivants :
(1) les homopolymères ou copolymères d'esters ou d'amides acryliques ou méthacryliques, à fonctions aminées, comportant au moins un des motifs de formules suivantes : 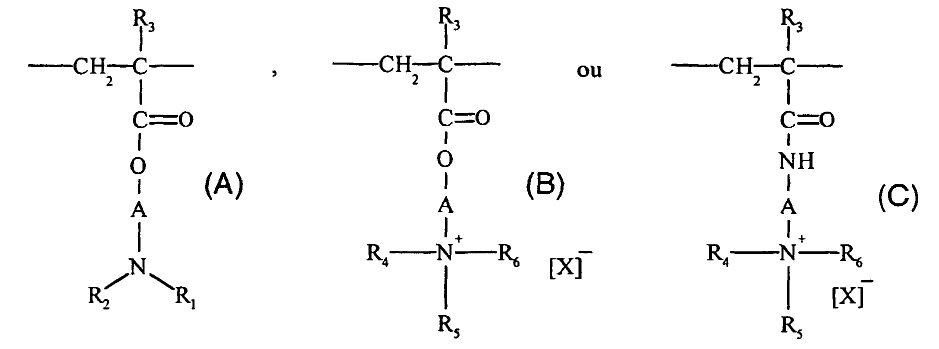 dans lesquelles:
   R₁ et R₂, identiques ou différents, représentent chacun un atome d'hydrogène ou un groupe alkyle ayant de 1 à 6 atomes de carbone ;
   R₃ désigne un atome d'hydrogène ou un groupe CH₃;
   A est un groupe alkyle linéaire ou ramifié, comportant de 1 à 6 atomes de carbone ou un groupe hydroxyalkyle comportant de 1 à 4 atomes de carbone ;
   R₄, R₅, R₆, identiques ou différents, représentent un groupe alkyle ayant de 1 à 18 atomes de carbone ou un groupe benzyle ;
   X désigne un anion méthosulfate ou un halogénure tel que chlorure ou bromure.
   Les copolymères de la famille (1) contiennent en outre un ou plusieurs motifs dérivant de comonomères pouvant être choisis dans la famille des acrylamides, méthacrylamides, diacétone-acrylamides, acrylamides et méthacrylamides substitués sur l'azote par des groupes alkyle inférieur (C₁₋₄), des groupes dérivés des acides acryliques ou méthacryliques ou de leurs esters, de vinyllactames tels que la vinylpyrrolidone ou le vinylcaprolactame, d'esters vinyliques.
   Ainsi, parmi ces copolymères de la famille (1), on peut citer :
   - les copolymères d'acrylamide et de méthacrylate de diméthylaminoéthyle quaternisés au sulfate de diméthyle ou avec un halogénure de diméthyle, tels que celui vendu sous la dénomination HERCOFLOC® par la société HERCULES,
   - les copolymères d'acrylamide et de chlorure de méthacryloyloxyéthyltriméthylammonium décrits, par exemple, dans la demande de brevet EP-A-080976 et vendus sous la dénomination BINA QUAT P 100 par la société CIBA GEIGY,
   - les copolymères d'acrylamide et de méthosulfate de méthacryloyloxyéthyltriméthylammonium, tels que celui vendu sous la dénomination RETEN par la société HERCULES,
   - les copolymères vinylpyrrolidone/acrylate ou méthacrylate de dialkylaminoalkyle quaternisés ou non, tels que les produits vendus sous la dénomination "GAFQUAT®" par la société ISP comme, par exemple, "GAFQUAT® 734" ou "GAFQUAT® 755", ou bien les produits dénommés "COPOLYMER® 845, 958 et 937". Ces polymères sont décrits en détail dans les brevets français Nos 2 077 143 et 2 393 573,
   - les polymères à chaîne grasse et à motif vinylpyrrolidone, tels que les produit vendus sous la dénomination Stylèze W20 et Stylèze W10 par la société ISP,
   - les terpolymères méthacrylate de diméthylaminoéthyle/vinylcaprolactame/vinylpyrrolidone tels que le produit commercialisé sous la dénomination GAFFIX® VC 713 par la société ISP, et
   - les copolymères vinylpyrrolidone/méthacrylamide de diméthylaminopropyle quaternisé tels que notamment le produit commercialisé sous la dénomination "GAFQUAT® HS 100" par la société ISP ;
(2) les polysaccharides cationiques, de préférence à ammonium quaternaire, tels que ceux décrits dans les brevets américains 3 589 578 et 4 031 307 tel que les gommes de guar contenant des groupements cationiques trialkylammonium. De tels produits sont commercialisés notamment sous les dénominations commerciales de JAGUAR C13 S, JAGUAR C 15, JAGUAR C 17 par la société MEYHALL;
(3) les copolymères quaternaires de vinylpyrrolidone et de vinylimidazole ;
(4) les chitosanes ou leurs sels ; les sels utilisables sont en particulier l'acétate, le lactate, le glutamate, le gluconate ou le pyrrolidone-carboxylate de chitosane.
   Parmi ces composés, on peut citer le chitosane ayant un taux de désacétylation de 90,5 % en poids vendu sous la dénomination KYTAN BRUT STANDARD par la société ABER TECHNOLOGIES, le pyrrolidone-carboxylate de chitosane commercialisé sous la dénomination KYTAMER® PC par la société AMERCHOL.
(5) les dérivés de cellulose cationiques tels que les copolymères de cellulose ou de dérivés de cellulose greffés avec un monomère hydrosoluble comportant un ammonium quaternaire, et décrits notamment dans le brevet US 4 131 576, tels que les hydroxyalkylcelluloses, comme les hydroxyméthyl-, hydroxyéthyl- ou hydroxypropyl-celluloses greffées notamment avec un sel de méthacryloyloxyéthyl-triméthylammonium, de méthacrylamidopropyl triméthylammonium, de diméthyl-diallylammonium.

Les produits commercialisés répondant à cette définition sont plus particulièrement les produits vendus sous la dénomination "CELQUAT L 200" et "CELQUAT H 100" par la Société National Starch.

Les polymères fixants cationiques peuvent également être choisis parmi les polymères siliconés greffés cationiques.

Les polymères fixants anioniques généralement utilisés sont des polymères comportant des groupements dérivés d'acide carboxylique, sulfonique ou phosphorique et ont une masse moléculaire moyenne en nombre comprise entre environ 500 et 5 000 000.

Les groupements carboxyliques sont apportés par des monomères mono- ou diacides carboxyliques insaturés tels que ceux répondant à la formule : 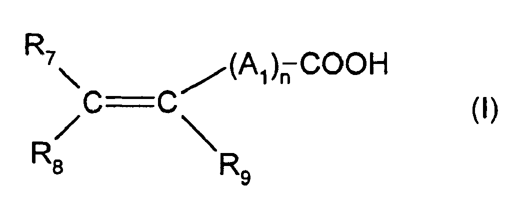 dans laquelle n est un nombre entier de 0 à 10, A, désigne un groupement méthylène, éventuellement relié à l'atome de carbone du groupement insaturé ou au groupement méthylène voisin lorsque n est supérieur à 1, par l'intermédiaire d'un hétéroatome tel que oxygène ou soufre, R₇ désigne un atome d'hydrogène, un groupement phényle ou benzyle, R₈ désigne un atome d'hydrogène, un groupement alkyle inférieur ou carboxyle, R₉ désigne un atome d'hydrogène, un groupement alkyle inférieur, un groupement -CH₂-COOH, phényle ou benzyle.

Dans la formule précitée, un groupement alkyle inférieur désigne de préférence un groupement ayant 1 à 4 atomes de carbone et en particulier, les groupements méthyle et éthyle.

Les polymères fixants anioniques à groupements carboxyliques préférés selon l'invention sont :
A) Les homo- ou copolymères d'acide acrylique ou méthacrylique ou leurs sels et en particulier les produits vendus sous les dénominations VERSICOL® E ou K par la société ALLIED COLLOID, et ULTRAHOLD® par la société BASF, les copolymères d'acide acrylique et d'acrylamide vendus sous la forme de leurs sels de sodium sous les dénominations RETEN 421, 423 ou 425 par la Société HERCULES, les sels de sodium des acides polyhydroxycarboxyliques.
B) Les copolymères d'acide acrylique ou méthacrylique avec un monomère monoéthylénique tel que l'éthylène, le styrène, les esters vinyliques, les esters d'acide acrylique ou méthacrylique, éventuellement greffés sur un polyalkylène-glycol tel que le polyéthylène-glycol, et éventuellement réticulés. De tels polymères sont décrits en particulier dans le brevet français n° 1 222 944 et la demande allemande n° 2 330 956, les copolymères de ce type comportant dans leur chaîne un motif acrylamide éventuellement N-alkylé et/ou hydroxyalkylé tels que décrits notamment dans les demandes de brevets luxembourgeois n°s 75370 et 75371 ou proposés sous la dénomination QUADRAMER par la Société AMERICAN CYANAMID. On peut également citer les copolymères d'acide acrylique et de méthacrylate d'alkyle en C₁-C₄ et les terpolymères de vinylpyrrolidone, d'acide acrylique et de méthacrylate d'alkyle en C,-C₂₀, par exemple, de lauryle, tels que celui commercialisé par la société ISP sous la dénomination ACRYLIDONE® LM et les terpolymères acide méthacrylique/acrylate d'éthyle/acrylate de tertiobutyle tels que le produit commercialisé sous la dénomination LUVIMER® 100 P par la société BASF.
   On peut aussi citer les copolymères acide méthacrylique/acide acrylique/acrylate d'éthyle/méthacrylate de méthyle en dispersion aqueuse, commercialisé sous la dénomination AMERHOLD® DR 25 par la société AMERCHOL.
C) Les copolymères d'acide crotonique, tels que ceux comportant dans leur chaîne des motifs acétate ou propionate de vinyle, et éventuellement d'autres monomères tels que les esters allylique ou méthallylique, éther vinylique ou ester vinylique d'un acide carboxylique saturé, linéaire ou ramifié, à longue chaîne hydrocarbonée, comme ceux comportant au moins 5 atomes de carbone, ces polymères pouvant éventuellement être greffés ou réticulés, ou encore un autre monomère ester vinylique, allylique ou méthallylique d'un acide carboxylique α- ou β-cyclique. De tels polymères sont décrits entre autres dans les brevets français n^{os} 1 222 944, 1 580 545, 2 265 782, 2 265 781, 1 564 110 et 2 439 798. Des produits commerciaux entrant dans cette classe sont les résines 28-29-30, 26-13-14 et 28-13-10 commercialisées par la société National Starch.
D) Les copolymères d'acides ou d'anhydrides carboxyliques monoinsaturés en C₄-C₈ choisis parmi :
   - les copolymères comprenant (i) un ou plusieurs acides ou anhydrides maléique, fumarique, itaconique et (ii) au moins un monomère choisi parmi les esters vinyliques, les éthers vinyliques, les halogénures vinyliques, les dérivés phénylvinyliques, l'acide acrylique et ses esters, les fonctions anhydrides de ces copolymères étant éventuellement monoestérifiées ou monoamidifiées. De tels polymères sont décrits en particulier dans les brevets US n^{os} 2 047 398, 2 723 248, 2 102 113, le brevet GB n° 839 805. Des produits commerciaux sont notamment ceux vendus sous les dénominations GANTREZ® AN ou ES par la société ISP.
   - les copolymères comprenant (i) un ou plusieurs motifs anhydrides maléique, citraconique, itaconique et (ii) un ou plusieurs monomères choisis parmi les esters allyliques ou méthallyliques comportant éventuellement un ou plusieurs groupements acrylamide, méthacrylamide, α-oléfine, esters acryliques ou méthacryliques, acides acrylique ou méthacrylique ou vinylpyrrolidone dans leur chaîne, les fonctions anhydrides de ces copolymères étant éventuellement monoestérifiées ou monoamidifiées.
   Ces polymères sont par exemple décrits dans les brevets français n^{os} 2 350 384 et 2 357 241 de la demanderesse.
E) Les polyacrylamides comportant des groupements carboxylates.

Les homopolymères et copolymères comprenant des groupements sulfoniques sont des polymères comportant des motifs vinylsulfonique, styrène-sulfonique, naphtalène-sulfonique ou acrylamido-alkylsulfonique.

Ces polymères peuvent être notamment choisis parmi :
- les sels de l'acide polyvinylsulfonique ayant une masse moléculaire comprise entre environ 1 000 et 100 000, ainsi que les copolymères avec un comonomère insaturé tel que les acides acrylique ou méthacrylique et leurs esters, ainsi que l'acrylamide ou ses dérivés, les éthers vinyliques et la vinylpyrrolidone.
- les sels de l'acide polystyrène-sulfonique tels que les sels de sodium vendus par exemple sous les dénominations Flexan® 500 et Flexan® 130 par National Starch. Ces composés sont décrits dans le brevet FR 2 198 719.
- les sels d'acides polyacrylamide-sulfoniques tels que ceux mentionnés dans le brevet US 4 128 631, et plus particulièrement l'acide polyacrylamidoéthylpropane-sulfonique vendu sous la dénomination COSMEDIA POLYMER HSP 1180 par Henkel.

Comme autre polymère fixant anionique utilisable selon l'invention, on peut citer le polymère anionique séquencé branché vendu sous la dénomination FIXATE G100 par la société NOVEON.

Selon l'invention, les polymères fixants anioniques sont de préférence choisis parmi les copolymères d'acide acrylique tels que les terpolymères acide acrylique/acrylate d'éthyle/N-tertiobutylacrylamide vendus notamment sous la dénomination ULTRAHOLD® STRONG par la société BASF, les copolymères dérivés d'acide crotonique tels que les terpolymères acétate de vinyle/tertio-butylbenzoate de vinyle/acide crotonique et les terpolymères acide crotonique/acétate de vinyle/néododécanoate de vinyle vendus notamment sous la dénomination Résine 28-29-30 par la société NATIONAL STARCH, les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters tels que les copolymères méthylvinyléther/anhydride maléique monoestérifié vendus, par exemple, sous la dénomination GANTREZ® par la société ISP, les copolymères d'acide méthacrylique et de méthacrylate de méthyle vendus sous la dénomination EUDRAGIT® L par la société ROHM PHARMA, les copolymères d'acide méthacrylique et d'acrylate d'éthyle vendus sous la dénomination LUVIMER® MAEX ou MAE par la société BASF et les copolymères acétate de vinyle/acide crotonique vendus notamment sous la dénomination LUVISET CA 66 par la société BASF et les copolymères acétate de vinyle/acide crotonique greffés par du polyéthylèneglycol vendus sous la dénomination ARISTOFLEX® A par la société BASF, le polymère vendu sous la dénomination FIXATE G100 par la société NOVEON.

Parmi les polymères fixants anioniques cités ci-dessus, on préfère plus particulièrement utiliser dans le cadre de la présente invention les copolymères méthylvinyléther/anhydride maléique monoestérifiés vendus sous la dénomination GANTREZ® ES 425 par la société ISP, les terpolymères acide acrylique/acrylate d'éthyle/N-tertiobutylacrylamide vendus sous la dénomination ULTRAHOLD® STRONG par la société BASF, les copolymères d'acide méthacrylique et de méthacrylate de méthyle vendus sous la dénomination EUDRAGIT® L par la société ROHM PHARMA, les terpolymères acétate de vinyle/tertio-butylbenzoate de vinyle/acide crotonique et les terpolymères acide crotonique/acétate de vinyle/ néododécanoate de vinyle vendus sous la dénomination Résine 28-29-30 par la société NATIONAL STARCH, les copolymères d'acide méthacrylique et d'acrylate d'éthyle vendus sous la dénomination LUVIMER® MAEX OU MAE par la société BASF, les terpolymères vinylpyrrolidone/acide acrylique/méthacrylate de lauryle vendus sous la dénomination ACRYLIDONE® LM par la société ISP, le polymère vendu sous la dénomination FIXATE G100 par la société NOVEON.

Les polymères fixants amphotères utilisables conformément à l'invention peuvent être choisis parmi les polymères comportant des motifs B et C répartis statistiquement dans la chaîne polymère où B désigne un motif dérivant d'un monomère comportant au moins un atome d'azote basique et C désigne un motif dérivant d'un monomère acide comportant un ou plusieurs groupements carboxyliques ou sulfoniques, ou bien B et C peuvent désigner des groupements dérivant de monomères zwittérioniques de carboxybétaïnes ou de sulfobétaïnes;

B et C peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un groupe hydrocarboné, ou bien B et C font partie d'une chaîne d'un polymère à motif éthylène-α,β-dicarboxylique dont l'un des groupements carboxyliques a été amené à réagir avec une polyamine comportant un ou plusieurs groupements amine primaire ou secondaire.

Les polymères fixants amphotères répondant à la définition donnée ci-dessus plus particulièrement préférés sont choisis parmi les polymères suivants :
(1) les copolymères à motifs vinyliques acides et à motifs vinyliques basiques, tels que ceux résultant de la copolymérisation d'un monomère dérivé d'un composé vinylique portant un groupement carboxylique tel que plus particulièrement l'acide acrylique, l'acide méthacrylique, l'acide maléïque, l'acide alpha-chloracrylique, et d'un monomère basique dérivé d'un composé vinylique substitué contenant au moins un atome basique, tel que plus particulièrement les méthacrylate et acrylate de dialkylaminoalkyle, les dialkylaminoalkyl-méthacrylamide et acrylamide. De tels composés sont décrits dans le brevet américain n° 3 836 537.
(2) les polymères comportant des motifs dérivant :
   a) d'au moins un monomère choisi parmi les acrylamides ou les méthacrylamides substitués sur l'atome d'azote par un groupe alkyle,
   b) d'au moins un comonomère acide contenant un ou plusieurs groupements carboxyliques réactifs, et
   c) d'au moins un comonomère basique tel que des esters à substituants amine primaire, secondaire, tertiaire et quaternaire des acides acrylique et méthacrylique, et le produit de quaternisation du méthacrylate de diméthylaminoéthyle avec le sulfate de diméthyle ou diéthyle.
   Les acrylamides ou méthacrylamides N-substitués plus particulièrement préférés selon l'invention sont les composés dont les groupes alkyle comportent de 2 à 12 atomes de carbone, et plus particulièrement le N-éthylacrylamide, le N-tertiobutylacrylamide, le N-tertiooctylacrylamide, le N-octylacrylamide, le N-décylacrylamide, le N-dodécylacrylamide ainsi que les méthacrylamides correspondants.
   Les comonomères acides sont choisis plus particulièrement parmi les acides acrylique, méthacrylique, crotonique, itaconique, maléïque, fumarique ainsi que les monoesters d'alkyle ayant 1 à 4 atomes de carbone des acides ou des anhydrides maléïque ou fumarique.
   Les comonomères basiques préférés sont des méthacrylates d'aminoéthyle, de butylaminoéthyle, de N,N'-diméthylaminoéthyle, de N-tertio-butylaminoéthyle.
   On utilise particulièrement les copolymères dont la dénomination CTFA (4ème Ed., 1991) est Octylacrylamide/acrylates/butylaminoethyl methacrylate copolymer, tels que les produits vendus sous la dénomination AMPHOMER® ou LOVOCRYL® 47 par la société NATIONAL STARCH.
(3) les polyaminoamides réticulés et acylés partiellement ou totalement dérivant de poyaminoamides de formule générale : 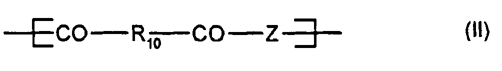 dans laquelle R₁₀ représente un groupe divalent dérivé d'un acide dicarboxylique saturé, d'un acide aliphatique mono ou dicarboxylique à double liaison éthylénique, d'un ester d'un alcanol inférieur ayant 1 à 6 atome de carbone de ces acides ou d'un groupe dérivant de l'addition de l'un quelconque desdits acides avec une amine bis-primaire ou bis-secondaire, et Z désigne un groupe dérivant d'une polyalkylène-polyamine bis-primaire, mono- ou bis-secondaire et de préférence représente :
   a) dans les proportions de 60 à 100 % en moles, le groupe 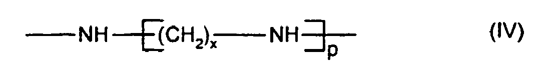 où x=2 et p=2 ou 3, ou bien x=3 et p=2
      ce groupe dérivant de la diéthylène-triamine, de la triéthylène-tétraamine ou de la dipropylène-triamine;
   b) dans les proportions de 0 à 40 % en moles, le groupe (IV) ci-dessus, dans lequel x=2 et p=1 et qui dérive de l'éthylène-diamine, ou le groupe dérivant de la pipérazine : 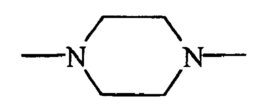
   c) dans les proportions de 0 à 20 % en moles, le groupe -NH-(CH₂)₆-NH- dérivant de l'hexaméthylènediamine, ces polyaminoamides étant réticulés par réaction d'addition d'un agent réticulant bifonctionnel choisi parmi les épihalohydrines, les diépoxydes, les dianhydrides, les dérivés bis-insaturés, au moyen de 0,025 à 0,35 mole d'agent réticulant par groupement amine du polyaminoamide, et acylés par action d'acide acrylique, d'acide chloracétique ou d'une alcane-sultone ou de leurs sels.
   Les acides carboxyliques saturés sont choisis de préférence parmi les acides ayant 6 à 10 atomes de carbone tels que les acides adipique, triméthyl-2,2,4-adipique et triméthyl-2,4,4-adipique, téréphtalique, les acides à double liaison éthylénique comme, par exemple, les acides acrylique, méthacrylique, itaconique.
   Les alcane-sultones utilisées dans l'acylation sont de préférence la propane- ou la butane-sultone, les sels des agents d'acylation sont de préférence les sels de sodium ou de potassium.
(4) les polymères comportant des motifs zwittérioniques de formule : 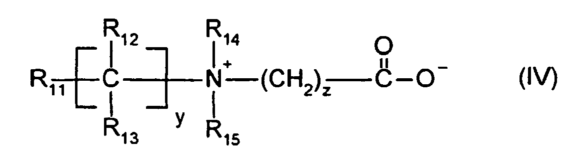 dans laquelle R₁₁ désigne un groupement insaturé polymérisable tel qu'un groupement acrylate, méthacrylate, acrylamide ou méthacrylamide, y et z représentent un nombre entier de 1 à 3, R₁₂ et R₁₃ représentent un atome d'hydrogène, un groupe méthyle, éthyle ou propyle, R₁₄ et R₁₅ représentent un atome d'hydrogène ou un groupe alkyle de telle façon que la somme des atomes de carbone dans R₁₄ et R₁₅ ne dépasse pas 10.
   Les polymères comprenant de tels motifs peuvent également comporter des motifs dérivés de monomères non zwittérioniques tels que l'acrylate ou le méthacrylate de diméthyl- ou diéthylaminoéthyle ou des acrylates ou méthacrylates d'alkyle, des acrylamides ou méthacrylamides, ou l'acétate de vinyle.
   A titre d'exemple, on peut citer les copolymères méthacrylate de méthyle/diméthyl-carboxyméthylammonio-éthylméthacrylate de méthyle, tels que le produit vendu sous la dénomination DIAFORMER Z301 par la société SANDOZ.
(5) les polymères dérivés du chitosane comportant des motifs monomères répondant aux formules suivantes : 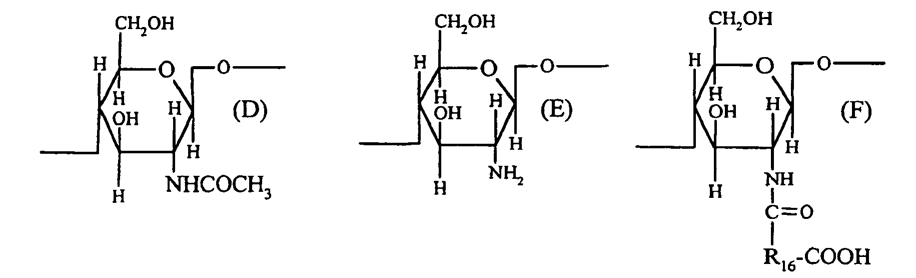 le motif (D) étant présent dans des proportions comprises entre 0 et 30%, le motif (E) dans des proportions comprises entre 5 et 50% et le motif (F) dans des proportions comprises entre 30 et 90%, étant entendu que dans ce motif (F), R₁₆ représente un groupe de formule : 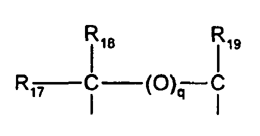 dans laquelle si q=0, R₁₇, R₁₈ et R₁₉, identiques ou différents, représentent chacun un atome d'hydrogène, un reste méthyle, hydroxyle, acétoxy ou amino, un reste monoalcoylamine ou un reste dialcoylamine éventuellement interrompus par un ou plusieurs atomes d'azote et/ou éventuellement substitués par un ou plusieurs groupes amine, hydroxyle, carboxyle, alcoylthio, sulfonique, un reste alcoylthio dont le groupe alcoyle porte un reste amino, l'un au moins des groupes R₁₇, R₁₈ et R₁₉ étant dans ce cas un atome d'hydrogène;
   ou si q=1, R₁₇, R₁₈ et R₁₉ représentent chacun un atome d'hydrogène, ainsi que les sels formés par ces composés avec des bases ou des acides.
(6) Les polymères répondant à la formule générale (V) sont, par exemple, décrits dans le brevet français 1 400 366 : 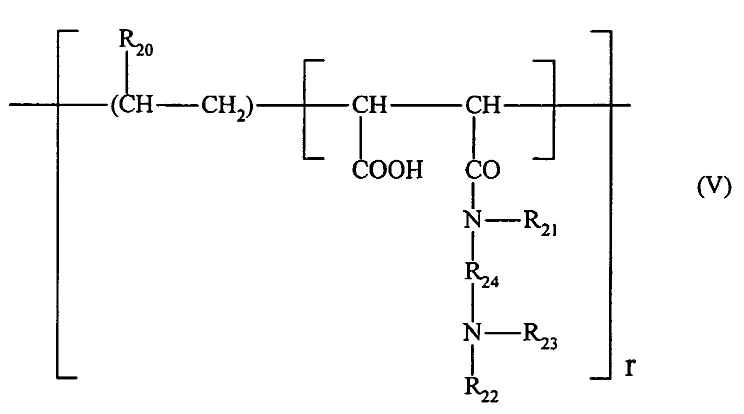 dans laquelle R₂₀ représente un atome d'hydrogène, un groupe CH₃O, CH₃CH₂O, phényle, R₂₁ désigne un atome d'hydrogène ou un groupe alkyle inférieur tel que méthyle, éthyle, R₂₂ désigne un atome d'hydrogène ou un groupe alkyle inférieur en C₁-C₆ tel que méthyle, éthyle, R₂₃ désigne un groupe alkyle inférieur en C₁-C₆ tel que méthyle, éthyle ou un groupe répondant à la formule : -R₂₄-N(R₂₂)₂, R₂₄ représentant un groupement -CH₂-CH₂-, -CH₂-CH₂-CH₂-, -CH₂-CH(CH₃)-, R₂₂ ayant les significations mentionnées ci-dessus.
(7) Les polymères dérivés de la N-carboxyalkylation du chitosane comme le N-carboxyméthyl-chitosane ou le N-carboxybutyl-chitosane, vendu sous la dénomination "EVALSAN" par la société JAN DEKKER.
(8) Les polymères amphotères du type -D-X-D-X choisis parmi:
   a) les polymères obtenus par action de l'acide chloracétique ou le chloracétate de sodium sur les composés comportant au moins un motif de formule :

      -D-X-D-X-D- (VI)

      où D désigne un groupe 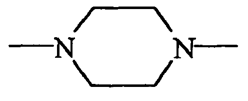 et X désigne le symbole E ou E', E ou E' identiques ou différents désignent un groupe bivalent qui est un groupe alkylène à chaîne droite ou ramifiée, comportant jusqu'à 7 atomes de carbone dans la chaîne principale non substituée ou substituée par des groupements hydroxyle et pouvant comporter en outre des atomes d'oxygène, d'azote, de soufre, 1 à 3 cycles aromatiques et/ou hétérocycliques ; les atomes d'oxygène, d'azote et de soufre étant présents sous forme de groupements éther, thioéther, sulfoxyde, sulfone, sulfonium, alkylamine, alcénylamine, des groupements hydroxyle, benzylamine, oxyde d'amine, ammonium quaternaire, amide, imide, alcool, ester et/ou uréthanne.
   b) Les polymères de formule :

      -D-X-D-X- (VI')

      où D désigne un groupe 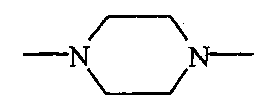 et X désigne le symbole E ou E' et au moins une fois E' ; E ayant la signification indiquée ci-dessus et E' est un groupe bivalent qui est un groupe alkylène à chaîne droite ou ramifiée, ayant jusqu'à 7 atomes de carbone dans la chaîne principale, substitué ou non par un ou plusieurs groupes hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisées par réaction avec l'acide chloracétique ou du chloracétate de soude.
(9) les copolymères alkyl(C₁-C₅)vinyléther/anhydride maléique modifiés partiellement par semiamidification avec une N,N-dialkylaminoalkylamine telle que la N,N-diméthylaminopropylamine ou par semiestérification avec un N,N-dialkylaminoalcanol. Ces copolymères peuvent également comporter d'autres comonomères vinyliques tels que le vinylcaprolactame.

Parmi les polymères fixants amphotères décrits ci-dessus, les plus particulièrement préférés selon l'invention sont ceux de la famille (3) tels que les copolymères dont la dénomination CTFA est Octylacrylamide/ acrylates/butylamino-ethylmethacrylate copolymer, tels que les produits vendus sous les dénominations AMPHOMER®, AMPHOMER® LV 71 ou LOVOCRYL® 47 par la société NATIONAL STARCH et ceux de la famille (4) tels que les copolymères méthacrylate de méthyle/diméthyl-carboxyméthylammonio-éthyl-méthacrylate de méthyle vendu par exemple sous la dénomination DIAFORMER® Z301 par la société SANDOZ.

Les polymères fixants non ioniques utilisables selon la présente invention sont choisis, par exemple, parmi :
- les polyalkyloxazolines ;
- les homopolymères d'acétate de vinyle;
- les copolymères d'acétate de vinyle tels que, par exemple, les copolymères d'acétate de vinyle et d'ester acrylique, les copolymères d'acétate de vinyle et d'éthylène, ou les copolymères d'acétate de vinyle et d'ester maléïque, par exemple, de maléate de dibutyle ;
- les homopolymères et copolymères d'esters acryliques tels que, par exemple, les copolymères d'acrylates d'alkyle et de méthacrylates d'alkyle tels que les produits proposés par la société ROHM & HAAS sous les dénominations PRIMAL® AC-261 K et EUDRAGIT® NE 30 D, par la société BASF sous la dénomination 8845, par la société HOECHST sous la dénomination APPRETAN® N9212 ;
- les copolymères d'acrylonitrile et d'un monomère non ionique choisis, par exemple, parmi le butadiène et les (méth)acrylates d'alkyle; on peut citer les produits proposés sous la dénomination CJ 0601 B par la société ROHM & HAAS;
- les homopolymères de styrène;
- les copolymères de styrène comme, par exemple, les copolymères de styrène et de (méth)acrylate d'alkyle tels que les produits MOWILITH® LDM 6911, MOWILITH® DM 611 et MOWILITH® LDM 6070 proposés par la société HOECHST, les produits RHODOPAS® SD 215 et RHODOPAS® DS 910 proposés par la société RHONE POULENC ; les copolymères de styrène, de méthacrylate d'alkyle et d'acrylate d'alkyle; les copolymères de styrène et de butadiène ; ou les copolymères de styrène, de butadiène et de vinylpyridine ;
- les polyamides ;
- les homopolymères de vinyllactame différents des homopolymères de vinylpyrrolidone, tels que le polyvinylcaprolactame commercialisé sous la dénomination Luviskol® PLUS par la société BASF ; et
- les copolymères de vinyllactame tels qu'un copolymère poly(vinylpyrrolidone/vinyllactame) vendu sous le nom commercial Luvitec® VPC 55K65W par la société BASF, les copolymères poly(vinylpyrrolidone/acétate de vinyle) comme ceux commercialisés sous la dénomination PVPVA® S630L par la société ISP, Luviskol® VA 73, VA 64, VA 55, VA 37 et VA 28 par la société BASF ; et les terpolymères poly(vinylpyrrolidone/acétate de vinyle/propionate de vinyle) comme, par exemple, celui commercialisé sous la dénomination Luviskol® VAP 343 par la société BASF.

Les groupes alkyle des polymères non ioniques mentionnés ci-dessus ont, de préférence, de 1 à 6 atomes de carbone.

Selon l'invention, on peut également utiliser des polymères fixants de type siliconés greffés, comprenant une partie polysiloxane et une partie constituée d'une chaîne organique non siliconée, l'une des deux parties constituant la chaîne principale du polymère et l'autre étant greffée sur ladite chaîne principale.

Ces polymères sont par exemple décrits dans les demandes de brevet EP-A-0 412 704, EP-A-0 412 707, EP-A-0 640 105 et WO 95/00578, EP-A-0 582 152 et WO 93/23009 et les brevets US 4,693,935, US 4,728,571 et US 4,972,037.

Ces polymères peuvent être amphotères, anioniques ou non ioniques, et ils sont de préférence anioniques ou non ioniques.

De tels polymères sont, par exemple, les copolymères susceptibles d'être obtenus par polymérisation radicalaire à partir du mélange de monomères formé :
a) de 50 à 90 % en poids d'acrylate de tertiobutyle,
b) de 0 à 40 % en poids d'acide acrylique,
c) de 5 à 40 % en poids d'un macromère siliconé de formule 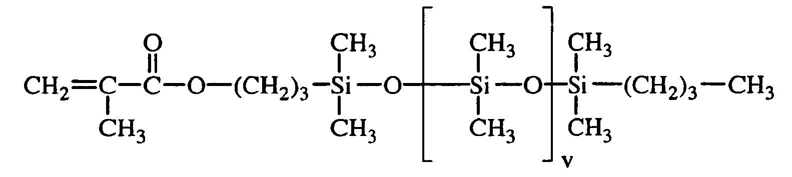
où v est un nombre allant de 5 à 700, les pourcentages en poids étant calculés par rapport au poids total des monomères.

D'autres exemples de polymères siliconés greffés sont notamment des polydiméthylsiloxanes (PDMS) sur lesquels sont greffés, par l'intermédiaire d'un chaînon de raccordement de type thiopropylène, des motifs polymères mixtes du type poly(acide (méth)acrylique) et du type poly((méth)acrylate d'alkyle), et des polydiméthylsiloxanes (PDMS) sur lesquels sont greffés, par l'intermédiaire d'un chaînon de raccordement de type thiopropylène, des motifs polymères du type poly(méth)acrylate d'isobutyle.

Comme autre type de polymères fixants siliconés, on peut citer le produit Luviflex® Silk commercialisé par la société BASF.

On peut également utiliser comme polymères fixants, des polyuréthanes fonctionnalisés ou non, siliconés ou non, cationiques, non-ioniques, anioniques ou amphotères, ou leurs mélanges.

Les polyuréthanes particulièrement visés par la présente invention sont ceux décrits dans les demandes EP 0 751 162, EP 0 637 600, EP 0 648 485 et FR 2 743 297 dont la demanderesse est titulaire, ainsi que dans les demandes EP 0 656 021 et WO 94/03510 de la société BASF, et EP 0 619 111 de la société National Starch.

Comme polyuréthanes convenant particulièrement bien dans la présente invention, on peut citer les produits commercialisés sous les dénominations LUVISET PUR® et LUVISET® Si PUR par la société BASF.

Les agents conditionneurs peuvent être choisis parmi les huiles, naturelles ou synthétiques, siliconées ou non, fluorées ou non, linéaires ou ramifiées. Parmi les huiles siliconées, on peut utiliser des huiles volatiles ou non, cycliques ou non, organomodifiées ou non. Ils peuvent être également choisis parmi les cires, naturelles ou synthétiques, siliconées ou non, fluorées ou non. Parmi ces cires, les cires d'origine végétale et les céramides sont préférées. Les agents conditionneurs peuvent également être choisis parmi les tensioactifs cationiques portant au moins une chaîne grasse et comprenant éventuellement une fonction ester.

Les polymères conditionneurs peuvent être choisis parmi les polymères cationiques et en particulier les polymères cationiques dérivés de guar et de cellulose et les cyclopolymères comportant comme monomère le chlorure de diméthyldialkylammonium, ainsi que parmi les polymères amphotères et notamment les copolymères acide acrylique / chlorure de diméthyldialkylammonium.

Le gel utilisé dans l'invention est un gel comprenant au moins un réseau réticulé de particules de polymère réticulé. Il est formé de particules de polymère reliées entre elles, ces particules étant elles-mêmes formées de polymère réticulé. Par « particules de polymère réticulé », on entend des particules pouvant être constituées d'un ou de plusieurs polymères réticulés. Le gel peut être formé de la façon suivante : formation des particules de polymère, réticulation du polymère formant les particules, puis formation du réseau réticulé de particules en réticulant les particules entre elles.

Les chaînes de polymère peuvent être réticulées entre elles par des fonctions présentes sur le polymère ou par des agents de réticulation pouvant être choisis parmi les divinyléthers de diols aliphatiques et les divinyléthers de diols aliphatiques et les divinyléthers de polyéthylèneglycols et aussi parmi les 1,7-octadiène, 1,9-décadiène, divinylbenzène, N,N'-bis-méthylène acrylamide, diacrylate de polyéthylène glycol ou de propylène glycol, triacrylate de triméthylolpropane, et les alcools polyhydriques esterifiés par l'acide acrylqiue. On utilise plus particulièrement le 1,9-décadiène.

Les chaînes de polymères peuvent ainsi former des particules de taille micrométrique ou nanométrique. Les particules peuvent être pleines ou creuses, et de forme sphérique, elliptique, ou encore polyhexagonale.

Les fonctions présentes sur les chaînes de polymère sont de préférence suffisamment nombreuses pour assurer la réticulation des chaînes de polymère et former des particules et pour qu'il y ait des fonctions encore disponibles à la surface des particules pour pouvoir, dans un deuxième temps, réticuler les particules entre elles et former ainsi le réseau de particules.

Avantageusement, le polymère utilisé pour former le réseau réticulé de particules réticulées est un polymère répondant à un stimulus extérieur. On entend par « polymère répondant à un stimulus extérieur », un polymère qui change ses propriétés de façon réversible lorsqu'il est soumis à un stimulus tel que la température, les rayons UV, le pH, un champ électrique, un champ magnétique, un changement de solvant ou encore du degré de salinité. De préférence, on attend que le polymère se rétracte de façon réversible une fois soumis à l'un de ces stimulus.

Le polymère répondant à une élévation de température peut être chois parmi les polyacrylamides, les polyvinylcaprolactames, les méthyle polyvinyléthers, les 3-hydroxy polypropylacrylates, les polyvinylacétates, les polyvinyloxazolines, les polyéthylène oxydes, les polypropylène oxydes, les polyalkylacrylamides, les polyhydroxypropyl celluloses, les polyvinylalcools, et leurs copolymères ; les polymères répondant au changement de pH peuvent être ceux cités dans les références Y.Ito, S.Kotera, M.Inaba, K.Kono, Y.Imanishi, Polymer, 31 (1990) 2157-61 et Y.Ito, M.Inaba, DJ.Chung, Y.Imanishi, Macromolecules, 25 (1992) 7313-16 ; les polymères changeant leur conformation sous l'influence de rayons UV peuvent être choisis parmi les polyacrylamides ayant de faibles quantités de triphénylméthane leucohydroxydes ou des groupements leucocyanides (M.Irie, Applied Photochromic polymer systems., (1992) 174-206, M.Irie, Adv. Polym. Sci., 94 (1990) 27-67) ; les polymères répondant aux changements de solvant peuvent être choisis parmi ceux cités dans les références M.Ilavsky, J.Hronz, J.Stejskal, K.Boudal, Macromolecule, 17 (1984) 2668 et T.Tanaka, Sci. Am., 244 (1981) 110 ; les polymères répondant aux champs électriques peuvent être choisis parmi ceux cités dans la référence T.Tanaka, I.Nishio, S.T.Sun, S.V.Nishio, Science, 218 (1973) 467.

Plus particulièrement, on peut mettre en oeuvre le procédé suivant : on disperse un polymère répondant au stimulus température dans un solvant, et on chauffe à une température supérieure à la température limite de transition pour une rétractation, de façon à ce que les chaînes de polymère se rétractent et forment des particules de polymère. On réticule ensuite le polymère à l'intérieur des particules à l'aide de fonctions présentes sur le polymère ou à l'aide d'au moins un agent de réticulation. Enfin, on procède à la réticulation des particules de polymère entre elles, à l'aide d'un agent réticulant, pour former le réseau réticulé de particules de polymère réticulé.

Les particules de polymère réticulé peuvent être formées à partir d'un ou plusieurs polymères.

La taille des particules peut être comprise entre 1 et 100 nm, et de préférence entre 1 et 50 nm. La taille de toutes les particules peuvent être identiques ou non.

Le gel contient au moins un réseau de particules réticulées. Si le gel contient plusieurs réseaux, ceux-ci seront imbriqués les uns dans les autres. Chaque réseau peut contenir un ou plusieurs types de particules de polymère(s), avec un ou plusieurs types de polymères pour chaque particule.

Les particules de polymère réticulé peuvent être présentes dans une quantité comprise entre 0,4 et 60%, de préférence entre 1 et 30%, et encore de préférence entre 2 et 20% du poids total de la composition. La taille du ou des réseau(x), c'est-à-dire l'espacement moyen entre les particules réticulées d'un même réseau, peut être comprise entre 50 et 1000 nm, et de préférence entre 50 et 500 nm. Les particules sont de préférence liées entre elles par des liens covalents entre des groupes fonctionnels situés à la surface des particules. Les liens de réticulation entre les particules sont dégradables ou non dégradables et peuvent être coupés par un stimulus extérieur ou non.

Le gel utilisé dans les compositions selon l'invention a une conformation relativement stable. La distance entre les particules de polymère(s) n'est pas perturbée par l'ajout d'additifs, le changement de solvant ou par une agitation mécanique, en particulier pour les polymères répondant à la température et/ou aux rayons UV. Dans le cas où la taille et l'espacement entre les particules sont bien maîtrisés, c'est-à-dire si les particules sont mono disperses, et si l'espacement est régulier entre les particules (cas des polymères répondant à un stimulus extérieur), le réseau diffracte la lumière et donne au gel une couleur opale. La couleur dépend en effet de l'espacement entre les particules.

Pour un polymère donné, la taille des particules dépend du type de polymère, du mode de synthèse et du rapport monomère/agent de réticulation utilisé pour la synthèse. L'espacement moyen entre les différentes particules du réseau dépend du mode de réticulation utilisé et dépend notamment de la proportion particules de polymère/agents de réticulation si des agents de réticulation sont utilisés.

Avantageusement, le réseau réticulé de particules de polymère réticulé répond à un stimulus extérieur comme les polymères décrits précédemment. Le stimulus extérieur est de préférence la température ou les rayons UV.

Dans ce cas particulier, la cinétique de rétractation du gel utilisé dans les compositions selon l'invention est asymétrique par rapport à la cinétique de rétractation d'hydrogels classiques : la vitesse de rétractation du gel est plus rapide que celle d'un gel classique, alors que la vitesse d'expansion du gel (retour à l'état initial lorsqu'il n'est plus soumis à une source de chaleur) n'est pas significativement plus élevée que celle d'un gel classique.

La synthèse des gels de l'invention peut s'effectuer en deux étapes : (1) formation de particules de polymère réticulé, puis (2) assemblage des particules dans un réseau à trois dimensions, et formation de liaisons covalentes entre les particules via les groupes fonctionnels à leur surface. Ce procédé est décrit dans la demande WO 03/022910.

Le composé cosmétiquement actif peut être présent dans la composition à des teneurs comprises entre 0,0001 et 60%, de préférence entre 0,01 et 30%, et encore de préférence entre 0,1 et 15% en poids du poids total de la composition.

Le milieu cosmétiquement acceptable peut contenir de l'eau ou un ou plusieurs solvants cosmétiquement acceptables tels que des alcools ou des mélanges eau-solvant(s), ces solvants étant de préférence des alcools en C2-C4, ou peut contenir un mélange d'eau et d'un ou plusieurs solvants cosmétiquement acceptables.

Parmi ces alcools, on peut citer l'éthanol, l'isopropanol. l'éthanol est particulièrement préféré.

On peut également utiliser comme solvant des alcanes en C5-C10, comme l'acétone, la méthyléthylcétone, l'acétate de méthyle, l'acétate de butyle, l'acétate d'éthyle, le diméthoxyéthane, le diéthoxyéthane. On peut également utiliser des alcools gras, des polyols modifiés ou non, comme le glycérol, le propylène glycol, les polyéthylène glycols, des silicones volatiles ou non, des huiles minérales, organiques ou végétales, des cires des acides gras, et leurs mélanges.

Avantageusement, le milieu cosmétiquement acceptable contient de l'eau.

Les compositions de l'invention peuvent être utilisées pour la fabrication de nombreux produits cosmétiques, en particulier capillaires, comme par exemple, des produits pour la fixation et/ou le maintien des cheveux, des produits de conditionnement, comme des formulations de brillance ou encore des produits de soin des cheveux.

Ces compositions peuvent être conditionnées sous diverses formes, notamment dans des flacons pompes ou dans des récipients aérosols, afin d'assurer une application de composition sous forme vaporisée ou sous forme de mousse. De telles formes de conditionnement sont indiquées, par exemple, lorsque l'on souhaite obtenir un spray, une laque, ou une mousse pour la fixation ou le traitement des cheveux. Les compositions conformes à l'invention peuvent également se présenter sous la forme de crèmes, de gels, d'émulsions, de lotions, ou de cires.

La composition selon l'invention est notamment est un gel, utilisable dans le domaine cosmétique pour le maquillage du visage et/ou du corps. En particulier, ce gel peut constituer un fond de teint ou un fard à joues, appelé aussi blush. L'invention se rapporte également à un procédé de maquillage consistant à appliquer ce gel sur le visage. On utilise parfois pour le maquillage de la peau des gels aqueux constitués d'une phase hydrophile comportant des agents gélifiants formés essentiellement de polymères hydrophiles. Ces gels aqueux ne comportent pas de phase grasse. Ils peuvent en outre être colorés par incorporation de colorants et/ou de pigments hydrophiles et apparaissent opaques ou translucides. Ces gels servent, en particulier, à la préparation de fonds de teint ou de blush. Ces gels aqueux colorés translucides sont connus pour leur apport de « bonne mine » et leur fraîcheur à l'application ; l'utilisateur de tels gels n'a pas l'impression d'être maquillé. Toutefois, ces gels ont l'inconvénient de procurer un inconfort aux personnes à peau sèche en raison de l'absence de corps gras et de provoquer un effet collant et tenseur de la peau du fait de la présence d'agents gélifiants. En outre, l'application de ces gels est souvent difficile du fait d'un manque d'effet glissant et d'un séchage trop rapide ; ces gels confèrent à la peau une hétérogénéité de maquillage, c'est-à-dire des zones claires et foncées. Pour remédier à l'inconfort de ces gels, on est conduit à incorporer dans ces derniers des hydratants comme par exemple des polyols, tels que la glycérine, mais l'effet collant conféré à la peau s'en trouve alors accentué. Ces inconvénients rendent l'utilisation des gels malaisée, voire rédhibitoire. Les gels comprenant une composition selon l'invention sont au contraire d'application aisée, glissent et s'étalent bien, sont non collants, ne sèchent pas trop vite et confèrent à la peau un maquillage homogène. Ils sont également particulièrement consistants, stables, d'aspect non grumeleux, de sensation agréable au toucher et sont compatibles avec les électrolytes et/ou les alcools primaires.

Lorsque la composition selon l'invention est conditionnée sous forme d'aérosol en vue d'obtenir une laque ou une mousse, elle comprend au moins un agent propulseur qui peut être choisi parmi les hydrocarbures volatils tels que le n-butane, le propane, l'isobutane, le pentane, un hydrocarbure halogéné et leurs mélanges. On peut également utiliser en tant qu'agent propulseur le gaz carbonique, le protoxyde d'azote, le diméthyléther (DME), l'azote, l'air comprimé. On peut aussi utiliser des mélanges de propulseurs. De préférence, on utilise le diméthyléther.

Avantageusement, l'agent propulseur est présent à une concentration comprise entre 5 et 90% en poids par rapport au poids total au poids total de la composition dans le dispositif aérosol, et plus particulièrement à une concentration comprise entre 10 et 60%.

L'invention a également pour objet un gel cosmétique, comprenant une composition selon l'une quelconque des revendications 1 à 27.

L'invention a enfin pour objet l'utilisation d'un gel comprenant au moins un réseau réticulé de particules de polymère réticulé pour la fabrication d'une composition cosmétique, notamment capillaire ou de maquillage.

La composition cosmétique peut être destinée au maintien et/ou à la mise en forme de fibres kératiniques, en particulier des cheveux. Il est bien évidemment possible d'utiliser les compositions de l'invention pour le maintien et/ou la mise en forme de fibres kératiniques, en particulier des cheveux.

L'invention a également pour objet un procédé cosmétique capillaire comprenant l'application sur les cheveux d'une composition selon l'invention.

Dans un mode de réalisation préféré du procédé selon l'invention, le procédé comprend en outre, simultanément ou de préférence consécutivement à l'application de la composition sur les cheveux, l'exposition des cheveux à la chaleur.

Les exemples suivants sont destinés à illustrer l'invention.

### Exemple 1

### Composition 1 selon l'invention

| | |
|---|---|
| Microgel de N isopropylacrylamide (NIPAM) | 95.0% |
| Polymère fixant (1) | 0.5% |
| Eau | 4.5% |
| (1) polymère commercialisé sous la dénomination FIXATE G100 par la société NOVEON | |

### Composition 2 (référence)

| | |
|---|---|
| Carbopol (1) | 0,3% |
| Polymère fixant (2) | 0,50% MA |
| Eau | 99,2% |
| MA : matière active | |
| (1) produit commercialisé sous la dénomination Synthalen K par la société NOVEON | |
| (2) polymère commercialisé sous la dénomination FIXATE G100 par la société NOVEON | |

Le microgel de NIPAM est obtenu selon le mode opératoire suivant : 1.62g de N isopropylacrylamide monomère + 0.165g 3-aminopropyl methacrylamide (7%) + 0.06g d'agent de réticulation classique comme le méthylbisacrylamide. Le mélange est chauffé à 53°C, on ajoute 0.056g de tensio-actif (DTAB) et 50 mg de persulfite de potassium comme initiateur, et le pH est ajusté à 5.4. Les particules sont formées après 2 heures de réaction. Le mélange est ensuite débarrassé du tensio-actif. On procède ensuite à une concentration du milieu puis à la réticulation du gel avec le même agent réticulant.

1g de composition à base de micro-gel de NIPAM (formule 1) est appliqué sur 2.5 g de cheveux européens.

1g de composition de référence (formule 2) est appliqué sur 2.5 g de cheveux châtains européens de même nature.

Après 2 minutes de pose, les cheveux sont séchés sous séchoir ou sous climason.

On soumet les mèches sur lesquelles les formules 1 et 2 ont été appliquées à des mouvements circulaires répétés. En comparant la tenue des mèches formés au bout d'une heure, d'une demi-journée de manière visuelle, on constate que la composition selon l'invention (composition 1) confère aux mèches une meilleure tenue et une meilleure rigidité.

### Exemple 2

### Composition 1 selon l'invention

| | |
|---|---|
| Microgel de N isopropylacrylamide (NIPAM) | 95.0% |
| Polymère fixant (1) | 0.5% |
| Eau | 4.5% |
| (1) polymère commercialisé sous la dénomination FIXATE G100 par la société NOVEON | |

### Composition 3 (référence)

| | |
|---|---|
| Poly N isopropylacrylamide non réticulé (1) | 0.3% |
| Polymère fixant (2) | 3.0% |
| Eau | 96.7% |
| (1) produit obtenu par polymérisation radicalaire en émulsion, comme par exemple décrit dans l'article NC Woodwurd, BZ Chowdhzy, MJ Snowden, SA Leharne, PC Griffiths, AL Winnington, Langmuir 19 (2003) 3202-3211 | |
| (2) polymère commercialisé sous la dénomination FIXATE G100 par la société NOVEON | |

1g de composition à base de micro-gel de NIPAM (mode de synthèse décrit dans l'exemple 1) est appliqué sur 2.5 g de cheveux européens. 1g de composition de référence 3 est appliqué sur 2.5 g de cheveux châtains européens de même nature. Après 2 minutes de pose, les cheveux sont séchés sous séchoir ou sous climason.

On soumet les mèches sur lesquelles les compositions 1 et 3 ont été appliquées à des mouvements circulaires répétés. En comparant la tenue des méchés formés au bout d'une heure, d'une demi-journée de manière visuelle, on constate que la composition selon l'invention (composition 1) confère aux méchés une meilleure tenue et une meilleure rigidité.

## Revendications

1. Utilisation d'un gel comprenant au moins un réseau réticulé de particules de polymère réticulé pour le traitement cosmétique des fibres kératiniques, et notamment des cheveux.

2. Utilisation selon la revendication 1, **caractérisée en ce que** le polymère répond à un stimulus extérieur.

3. Utilisation selon la revendication 2, **caractérisée en ce que** le stimulus extérieur est la température ou les rayons. UV.

4. Utilisation selon l'une quelconque des revendications. 1 à 3, **caractérisée en ce que** le composé cosmétiquement actif est choisi/parmi les agents réducteurs ou antioxydants, les agents oxydants, les silicones, les agents adoucissants, les agents anti-mousse, les agents hydratants, les agents émollients, les plastifiants, les filtres solaires, les colorants, les pigments, les charges, les argiles, les nacres ou agents nacrants, les parfums, les peptisants, les agents épaississants, les conservateurs, les polymères fixants, les vitamines ou provitamines, les agents conditionneurs, les polymères cationiques, les tensioactifs cationiques, les cires, les protéines.

5. Utilisation selon la revendication 4, **caractérisée en ce que** l'agent réducteur est choisi parmi NaBH₄, KBH₄, BH₃, NADPH, NADH, H₂, 9-BBN (9 borabicyclo[[3.3.1]nonane), LiAlH₄, NaAlEt₂H₂; Na₂S₂O₃, le diisoamylborane, l'acide ascorbique et les composés cycliques ou aliphatiques contenant le groupe chimique enediol -(OH)C=C(OH)- incluant les formes isomériques, leurs sels et leurs esters, l'hydroquinone, NaBH-(OAc)₃, l'acide formamidine sulfinique (FASA), l'acide N-phénylformadimine sulfinique, les phosphines, NaBH₃CN, Na₂S₂O₄, BMS (bismercaptoéthylsulfone), DTT (dithiothréitol), DMH (N,N'diméthyl-N,N'-bis(mercaptoacétyl)hydrazine), les thiols, les thiolates, les citrates, sulfites, bisulfites, monopersulfates et bisulfates de métaux alcalins, la protéine disulfure isomérase, la thiorédoxine, et les sulfinates.

6. Utilisation selon la revendication 4, **caractérisée en ce que** l'agent oxydant est choisi parmi l'eau oxygénée, les bromates et les persels.

7. Utilisation selon la revendication 4, **caractérisée en ce que** l'agent épaississant est choisi parmi la gomme de guar, la gomme de xanthane, la farine d'épicéa, la cellulose, leurs dérivés modifiés, les polymères synthétiques.

8. Utilisation selon la revendication 4, **caractérisée en ce que** les filtres solaires sont choisis parmi les anthranilates; les dérivés cinnamiques; les dérivés de dibenzoylméthane ; les dérivés salicyliques ; les dérivés du camphre; les dérivés de triazine, de préférence les dérivés de 1, 3, 5-triazine ; les dérivés de la benzophénone ; les dérivés de β-β'-diphénylacrylate; les dérivés de benzotriazole ; les dérivés de benzalmalonate ; des dérivés de benzimidazole ; les imidazolines ; les dérivés bis-benzoazolyle ; les dérivés de l'acide p-aminobenzoïque (PABA); les dérivés de méthylène bis-(hydroxyphényl benzotriazole) ; les polymères filtres et silicones filtres ; les dimères dérivés d'α-alkylstyrène ; les 4,4-diarylbutadiènes et leurs mélanges.

9. Utilisation selon la revendication 4, **caractérisée en ce que** le colorant est un colorant d'oxydation choisi parmi les bases d'oxydation et/ou les coupleurs.

10. Utilisation selon la revendication 9, **caractérisée en ce que** les bases d'oxydation sont choisies parmi les ortho- et para- phénylènediamines, les bases doubles, les ortho- et para- aminophénols, les bases hétérocycliques, ainsi que les sels d'addition de ces composés avec un acide.

11. Utilisation selon la revendication 10, **caractérisée en ce que** les bases hétérocycliques sont choisies parmi les dérivés pyridiniques, les dérivés pyrimidiniques, les dérivés pyrazoliques.

12. Utilisation selon la revendication 9, **caractérisée en ce que** les coupleurs sont choisis parmi les métaphénylènediamines, les méta-aminophénols, les métadiphénols, les coupleurs hérérocycliques, et les sels d'addition de ces composés avec un acide.

13. Utilisation selon la revendication 4, **caractérisée en ce que** le colorant est un colorant direct.

14. Utilisation selon la revendication 4, **caractérisée en ce que** les pigments sont choisis parmi le dioxyde de titane, les oxydes de zirconium, de zinc ou de cérium, les oxydes de fer ou de chrome, le violet de manganèse, le bleu outremer, l'hydrate de chrome, le bleu ferrique, le noir de carbone, les laques à base de carmin de cochenille, de baryum, strontium, calcium, aluminium.

15. Utilisation selon la revendication 4, **caractérisée en ce que** les nacres ou agents nacrants sont choisis parmi le mica recouvert de titane ou d'oxychlorure de bismuth, le micatitane avec des oxydes de fer, le mica titane avec du bleu ferrique ou de l'oxyde de chrome, le mica titane avec un pigment organique et les pigments nacrés à base d'oxychlorure de bismuth, les éthers ou esters organiques de insolubles.

16. Utilisation selon la revendication 4, **caractérisée en ce que** le ou les polymères fixants sont choisis parmi les polymères fixants cationiques, anioniques, amphotères, non ioniques et leurs mélanges.

17. Utilisation selon la revendication 16, **caractérisée en ce que** le ou les polymères fixants cationiques sont choisis parmi les homopolymères ou copolymères d'esters ou d'amides acryliques ou méthacryliques à fonctions aminées, les polysaccharides cationiques, les copolymères quaternaires de vinylpyrrolidone et de vinylimidazole, les polymères greffés cationiques et les chitosanes.

18. Utilisation selon la revendication 16, **caractérisée en ce que** le ou les polymères fixants anioniques sont choisis parmi les homopolymères ou copolymères d'acide acrylique et méthacrylique ou leurs sels, copolymères d'acide crotonique, les copolymères d'acides ou d'anhydrides carboxyliques monoinsaturés en C₄-C₈, les polyacrylamides à groupements carboxylates, les homopolymères ou copolymères, à groupes sulfoniques, les polyuréthanes anioniques, et les polymères siliconés greffés anioniques.

19. Utilisation selon la revendication. 16, **caractérisée en ce que** le ou les polymères fixants amphotères sont choisis parmi les copolymères à motifs vinyliques acides et à motifs vinyliques basiques, les polyaminoamides réticulés et acylés, les polymères à motifs zwittérioniques, les polymères dérivés du chitosane, les copolymères alkyl(C₁-C₅)vinyléther/anhydride maléique modifiés, les polyuréthanes amphotères et les polymères siliconés greffés amphotères.

20. Utilisation selon la revendication 16, **caractérisée en ce que** le ou les polymères fixants non toniques sont choisis parmi les polyalkyloxazolines, les homopolymères et copolymères d'acétate de vinyle, les homopolymères et copolymères d'esters acryliques, les copolymères d'acrylonitrile, les homopolymères et copolymères de styrène, les polyamides, les homopolymères de vinyllactame différents des homopolymères de vinylpyrrolidone, les copolymères de vinyllactame, les polyuréthanes non ioniques, et les polymères siliconés greffés non ioniques.

21. Utilisation selon la revendication 4, **caractérisée en ce que** l'agent conditionneur est choisi parmi les huiles naturelles les cires naturelles ou synthétiques, les tensioactifs cationiques et les polymères cationiques conditionneurs.

22. Utilisation selon l'une quelconque des revendications précédentes, **caractérisée en ce que** la taille des particules de polymère réticulé est comprise entre 1 et 100 nm, et de préférence entre 1 et 50 nm.

23. Utilisation selon l'une quelconque des revendications précédentes, **caractérisée en ce que** le réseau réticulé de particules de polymère réticulé est constitué de particules présentant un espacement moyen allant de 50 à 1000 nm, et de préférence de 50 à 500 nm.

24. Utilisation selon l'une quelconque des revendications précédentes pour le maintien et/ou la mise en forme des fibres kératiniques, en particulier des cheveux.

25. Utilisation d'un gel comprenant au moins un réseau réticulé de particules de polymère réticulé pour la fabrication d'une composition cosmétique capillaire.

26. Composition cosmétique, notamment capillaire, **caractérisée en ce qu**'elle, comprend, dans un milieu cosmétiquement acceptable, un gel comprenant au moins un réseau réticulé de particules de polymère réticulé, et au moins un composé cosmétiquement actif choisi parmi les agents réducteurs ou antioxydants, les agents oxydants, les silicones, les agents adoucissants, les agents anti-mousse, les agents hydratants, les agents émollients, les plastifiants, les filtres solaires, les colorants, les pigments, les charges, les argiles, les nacres ou agents nacrants, les parfums, les agents épaississants, les polymères fixants, les vitamines ou provitamines, les agents conditionneurs, les polymères cationiques, les tensioactifs cationiques, les cires.

27. Composition selon la revendication 26, **caractérisée en ce que** l'agent réducteur est choisi parmi NaBH₄, KBH₄, BH₃, NADPH, NADH, H₂, 9-BBN (9 borabicyclo[[3.3.1]nonane), LiAlH₄, NaAlEt₂H₂, Na₂S₂O₃, le diisoamylborane, l'acide ascorbique et les composés cycliques ou aliphatiques contenant le groupe chimique enediol -(OH)C=C(OH)- incluant les formes isomériques, leur sels et leurs esters, l'hydroquinone NaBH(OAc)₃, l'acide formamidine sulfinique (FASA), l'acide N-phénylformadimine sulfinique, les phosphines, NaBH₃CN, Na₂S₂O₄, BMS (bismercaptoéthylsulfone), DTT (dithiothréitol), DMH (N,N' diméthyl-N,N'-bis(mercaptoacétyl)hydrazine), les thiols, les thiolates, les citrates, sulfites, bisulfites, monopersulfates et bisulfates de métaux alcalins, la protéine disulfure isomérase, la thiorédoxine, et les sulfinates.

28. Composition selon la revendication 26, **caractérisée en ce que** l'agent oxydant est choisi parmi l'eau oxygénée, les bromates et les persels.

29. Composition selon la revendication 26, **caractérisée en ce que** l'agent épaississant est choisi parmi la gomme de guar, la gomme de xanthane, la farine d'épicéa, la cellulose, leurs dérivés modifiés, les polymères synthétiques.

30. Composition selon la revendication 26, **caractérisée en ce que** les filtres solaires sont choisis parmi les anthranilates; les dérivés cinnamiques; les dérivés de dibenzoylméthane; les dérivés, salicyliques ;les dérivés du camphre les dérivés de triazine, de préférence les dérivés de 1,3,5-triazine ; les dérivés de la benzophénone ; les dérivés de β-β'-diphénylacrylate ; les dérivés de benzotriazole ; les dérivés de benzalmalonate ; les dérivés de benzimidazole ; les imidazolines ; les dérivés bis-benzoazolyle ; les dérivés de l'acide p-aminobenzoïque (PABA); les dérivés de méthylène bis-(hydroxyphényl benzotriazole) ;les polymères filtres et silicones filtres ; les dimères dérivés d'α-alkylstyrène ; les 4,4-diarylbutadiènes et leurs mélanges.

31. Composition selon la revendication 26, **caractérisée en ce que** le colorant est un colorant d'oxydation choisi parmi les bases d'oxydation et/ou les coupleurs.

32. Composition selon la revendication 31, **caractérisée en ce que** les bases d'oxydation sont choisies parmi les ortho- et para- phénylènediamines, les bases doubles, les ortho- et para- aminophénols, les bases hétérocycliques, ainsi que les sels d'addition de ces composés avec un acide.

33. Composition selon la revendication 32, **caractérisée en ce que** les bases hétérocycliques sont choisies parmi les dérivés pyridiniques, les dérivés pyrimidiniques, les dérivés pyrazoliques.

34. Composition selon la revendication 31, **caractérisée en ce que** les coupleurs sont choisis parmi les métaphénylènediamines, les méta-aminophénols, les métadiphénols, les coupleurs hérérocycliques, et les sels d'addition de ces composés avec un acide.

35. Composition selon la revendication 26, **caractérisée en ce que** le colorant est un colorant direct.

36. Composition selon la revendication 26, **caractérisée en ce que** les pigments sont choisis parmi le dioxyde de titane, les oxydes de zirconium, de zinc ou de cérium, les oxydes de fer ou de chrome, le violet de manganèse, le bleu outremer, l'hydrate de chrome, le bleu ferrique, le noir de carbone, les laques à base de carmin de cochenille, de baryum, strontium, calcium, aluminium.

37. Composition selon la revendication 26, **caractérisée, en ce que** les nacres ou agents nacrants sont choisis parmi le mica recouvert de titane ou d'oxychlorure de bismuth, le micatitane avec des oxydes de fer, le mica titane avec du bleu ferrique ou de l'oxyde de chrome, le mica titane avec un pigment organique et les pigments nacrés à base d'oxychlorure de bismuth, les éthers ou esters organiques de insolubles.

38. Composition selon la revendication 26, **caractérisée en ce que** le ou les polymères fixants sont choisis parmi les polymères fixants cationiques, anioniques, amphotères, non ioniques et leurs mélanges.

39. Composition selon la revendication 38, **caractérisée en ce que** le ou les polymères fixants cationiques sont choisis parmi les homopolymères ou copolymères d'esters ou d'amides acryliques ou méthacryliques à fonctions aminées, les polysaccharides cationiques, les copolymères quaternaires de vinylpyrrolidone et de vinylimidazole, les polymères greffés cationiques et les chitosanes.

40. Composition selon la revendication 38, **caractérisée en ce que** le ou les polymères fixants anioniques sont choisis parmi les homopolymères ou copolymères d'acide acrylique et méthacrylique ou leurs sels, les copolymères d'acide crotonique, les copolymères d'acides ou d'anhydrides carboxyliques monoinsaturés en C₄-C₈, les polyacrylamides à groupements carboxylates, les homopolymères ou copolymères à groupes sulfoniques, les polyuréthanes anioniques, et les polymères siliconés greffés anioniques.

41. Composition selon la revendication 38, **caractérisée en ce que** le ou les polymères fixants amphotères sont choisis parmi les copolymères à motifs vinyliques acides et à motifs vinyliques basiques, les polyaminoamides réticulés, et acylés, les polymères à motifs zwittérioniques, les polymères dérivés du chitosane, les copolymères alkyl (C₁-C₅) vinyléther/anhydride maléique modifiés, les polyréthanes amphotères et les polymères siliconés greffés amphotères.

42. Composition selon la revendication 38, **caractérisée en ce que** le ou les polymères fixants non ioniques sont choisis parmi les polyalkyloxazolines, les homopolymères et copolymères d'acétate de vinyle, les homopolymères et copolymères d'esters acryliques, les copolymères d'acrylonitrile, les homopolymères et copolymères de styrène, les polyamides, les homopolymères de vinyllactame différents des homopolymères de vinylpyrrolidone, les copolymères de vinyllactame, les polyuréthanes non ioniques, et les polymères siliconés greffés non ioniques.

43. Composition selon la revendication 26, **caractérisée en ce que** l'agent conditionneur est choisi parmi les huiles naturelles ou synthétiques, les cires naturelles ou synthétiques, les tensioactifs cationiques et les polymères cationiques conditionneurs.

44. Composition selon l'une quelconque des revendications 26 à 43, **caractérisée en ce que** le ou les composés cosmétiquement actifs sont présents dans la composition à des teneurs comprises entre 0,0001 et 60%, de préférence entre 0,01 et 30%, et encore de préférence entre 0,1 et 15% en poids du poids total de la composition.

45. Composition selon l'une quelconque des revendications 26 à 44, **caractérisée en ce que** la taille des particules de polymère réticulé est comprise entre 1 et 100 nm, et de préférence entre 1 et 50 nm.

46. Composition selon l'une quelconque des revendications 26 à 45, **caractérisée en ce que** le réseau réticulé de particules de polymère, réticulé est constitué de particules présentant un espacement moyen allant de 50 à 1000 nm, et de préférence de 50 à 500 nm.

47. Composition selon l'une quelconque des revendications 26 à 46, **caractérisée en ce que** les particules de polymère réticulé sont présentes dans une quantité comprise entre 0,4 et 60%, de préférence entre 1 et 30%, et encore de préférence entre 2 et 20% du poids total de la composition.

48. Composition selon l'une quelconque des revendications 26 à 47, **caractérisée en ce que** le milieu cosmétiquement acceptable contient de l'eau ou un ou plusieurs solvants cosmétiquement acceptables, ou un mélange d'eau et d'un ou plusieurs solvants cosmétiquement acceptables.

49. Composition, selon l'une quelconque des revendications 26 à 48,
**caractérisée en ce que** le milieu cosmétiquement acceptable contient de l'eau.

50. Gel cosmétique, **caractérisé en ce qu**'il comprend une composition selon l'une quelconque des revendications 26 à 49.

51. Procédé cosmétique, capillaire, **caractérisé en ce qu**'il comprend l'application sur les cheveux d'une composition comprenant dans un milieu cosmétiquement acceptable, au moins un composé cosmétiquement actif et un gel comprenant au moins un réseau réticulé de particules de polymère réticulé.

52. Procédé, selon la revendication 51, **caractérisé en ce qu**'il comprend en outre, simultanément ou de préférence consécutivemet à l'application de la composition sur les cheveux, l'exposition des cheveux à la chaleur.

## Claims

1. Use of a gel comprising at least one crosslinked network of crosslinked polymer particles for the cosmetic treatment of keratin fibres, and especially the hair.

2. Use according to Claim 1, **characterized in that** the polymer responds to an external stimulus.

3. Use according to Claim 2, **characterized in that** the external stimulus is temperature or UV rays.

4. Use according to any one of Claims 1 to 3, **characterized in that** the cosmetically active compound is chosen from reducing agents or antioxidants, oxidizing agents, silicones, softeners, antifoams, moisturizers, emollients, plasticizers, sunscreens, dyes, pigments, fillers, clays, nacres or nacreous agents, fragrances, peptizers, thickeners, preserving agents, fixing polymers, vitamins or provitamins, conditioners, cationic polymers, cationic surfactants, waxes and proteins.

5. Use according to Claim 4, **characterized in that** the reducing agent is chosen from NaBH₄, KBH₄, BH₃, NADPH, NADH, H₂, 9-BBN (9 borabicyclo[[3.3.1]nonane), LiAlH₄, NaAlEt₂H₂, Na₂S₂O₃, diisoamylborane, ascorbic acid and cyclic or aliphatic compounds containing the enediol -(OH)C=C(OH)- chemical group, including the isomeric forms, and the salts and esters thereof, hydroquinone, NaBH(OAc)₃, formamidinesulfinic acid (FASA), N-phenylformadiminesulfinic acid, phosphines, NaBH₃CN, Na₂S₂O₄, BMS (bismercaptoethyl sulfone), DTT (dithiothreitol), DMH (N,N'-dimethyl-N,N'-bis(mercaptoacetyl)hydrazine), thiols, thiolates, citrates, sulfites, bisulfites, monopersulfates and bisulfates of alkali metals, the protein disulfide isomerase or thioredoxine type, and sulfinates.

6. Use according to Claim 4, **characterized in that** the oxidizing agent is chosen from aqueous hydrogen peroxide solution, bromates and persalts.

7. Use according to Claim 4, **characterized in that** the thickener is chosen from guar gum, xanthan gum, spruce meal, cellulose, modified derivatives thereof, and synthetic polymers.

8. Use according to Claim 4, **characterized in that** the sunscreens are chosen from anthranilates; cinnamic derivatives; dibenzoylmethane derivatives; salicylic derivatives; camphor derivatives; triazine derivatives, preferably 1,3,5-triazine derivatives; benzophenone derivatives; β,β'-diphenylacrylate derivatives; benzotriazole derivatives; benzalmalonate derivatives; benzimidazole derivatives; imidazolines; bis-benzazolyl derivatives; p-aminobenzoic acid (PABA) derivatives; methylenebis(hydroxyphenylbenzotriazole) derivatives; screening polymers and screening silicones; α-alkylstyrene-based dimers; 4,4-diarylbutadienes, and mixtures thereof.

9. Use according to Claim 4, **characterized in that** the dye is an oxidation dye chosen from oxidation bases and/or couplers.

10. Use according to Claim 9, **characterized in that** the oxidation bases are chosen from ortho- and para-phenylenediamines, double bases, ortho- and para-aminophenols and heterocyclic bases, and also the addition salts of these compounds with an acid.

11. Use according to Claim 10, **characterized in that** the heterocyclic bases are chosen from pyridine derivatives, pyrimidine derivatives and pyrazole derivatives.

12. Use according to Claim 9, **characterized in that** the couplers are chosen from meta-phenylenediamines, meta-aminophenols, meta-diphenols and heterocyclic couplers, and the addition salts of these compounds with an acid.

13. Use according to Claim 4, **characterized in that** the dye is a direct dye.

14. Use according to Claim 4, **characterized in that** the pigments are chosen from titanium dioxide, zirconium oxide, zinc oxide or cerium oxide, iron oxide or chromium oxide, manganese violet, ultramarine blue, chromium hydrate, ferric blue, carbon black, and lakes based on cochineal carmine or on barium, strontium, calcium or aluminium.

15. Use according to Claim 4, **characterized in that** the nacres or nacreous agents are chosen from mica coated with titanium or with bismuth oxychloride, titanium mica with iron oxides, titanium mica with ferric blue or chromium oxide, titanium mica with an organic pigment and nacreous pigments based on bismuth oxychloride, and insoluble organic ethers or esters.

16. Use according to Claim 4, **characterized in that** the fixing polymer(s) is (are) chosen from cationic, anionic, amphoteric and nonionic fixing polymers, and mixtures thereof.

17. Use according to Claim 16, **characterized in that** the cationic fixing polymer(s) is (are) chosen from homopolymers or copolymers of acrylic or methacrylic esters or amides containing amine functions, cationic polysaccharides, quaternary copolymers of vinylpyrrolidone and of vinylimidazole, cationic grafted polymers and chitosans.

18. Use according to Claim 16, **characterized in that** the anionic fixing polymers are chosen from homopolymers or copolymers of acrylic and methacrylic acid or salts thereof, crotonic acid copolymers, monounsaturated C₄-C₈ carboxylic acid or anhydride copolymers, polyacrylamides containing carboxylate groups, homopolymers or copolymers containing sulfonic groups, anionic polyurethanes and anionic grafted silicone polymers.

19. Use according to Claim 16, **characterized in that** the amphoteric fixing polymer(s) is (are) chosen from copolymers containing acidic vinyl units and basic vinyl units, crosslinked and acylated polyamino amides, polymers containing zwitterionic units, chitosan-based polymers, modified (C₁-C₅)alkyl vinyl ether/maleic anhydride copolymers, amphoteric polyurethanes and amphoteric grafted silicone polymers.

20. Use according to Claim 16, **characterized in that** the nonionic fixing polymer(s) is (are) chosen from polyalkyloxazolines, vinyl acetate homopolymers and copolymers, acrylic ester homopolymers and copolymers, acrylonitrile copolymers, styrene homopolymers and copolymers, polyamides, vinyllactam homopolymers other than vinylpyrrolidone homopolymers, vinyllactam copolymers, nonionic polyurethanes and nonionic grafted silicone polymers.

21. Use according to Claim 4, **characterized in that** the conditioner is chosen from natural or synthetic oils, natural or synthetic waxes, cationic surfactants and conditioning cationic polymers.

22. Use according to any one of the preceding claims, **characterized in that** the size of the crosslinked polymer particles is between 1 and 100 nm and preferably between 1 and 50 nm.

23. Use according to any one of the preceding claims, **characterized in that** the crosslinked network of crosslinked polymer particles consists of particles with a mean spacing of from 50 to 1000 nm and preferably from 50 to 500 nm.

24. Use according to any of the preceding claims for holding and/or shaping keratin fibres, in particular the hair.

25. Use of a gel comprising at least one crosslinked network of crosslinked polymer particles, for the manufacture of a cosmetic hair composition.

26. Cosmetic composition, especially a hair composition, **characterized in that** it comprises, in a cosmetically acceptable medium, a gel comprising at least one crosslinked network of crosslinked polymer particles, and at least one cosmetically active compound chosen from reducing agents or antioxidants, oxidizing agents, silicones, softeners, antifoams, moisturizers, emollients, plasticizers, sunscreens, dyes, pigments, fillers, clays, nacres or nacreous agents, fragrances, thickeners, fixing polymers, vitamins or provitamins, conditioners, cationic polymers, cationic surfactants, and waxes.

27. Composition according to Claim 26, **characterized in that** the reducing agent is chosen from NaBH₄, KBH₄, BH₃, NADPH, NADH, H₂, 9-BBN (9 borabicyclo[[3.3.1]nonane), LiAlH₄, NaAlEt₂H₂, Na₂S₂O₃, diisoamylborane, ascorbic acid and cyclic or aliphatic compounds containing the enediol -(OH)C=C(OH)- chemical group, including the isomeric forms, and the salts and esters thereof, hydroquinone, NaBH(OAc)₃, formamidinesulfinic acid (FASA), N-phenylformadiminesulfinic acid, phosphines, NaBH₃CN, Na₂S₂O₄, BMS (bismercaptoethyl sulfone), DTT (dithiothreitol), DMH (N,N'-dimethyl-N,N'-bis(mercaptoacetyl)hydrazine), thiols, thiolates, citrates, sulfites, bisulfites, monopersulfates and bisulfates of alkali metals, the protein disulfide isomerase or thioredoxine type, and sulfinates.

28. Composition according to Claim 26, **characterized in that** the oxidizing agent is chosen from aqueous hydrogen peroxide solution, bromates and persalts.

29. Composition according to Claim 26, **characterized in that** the thickener is chosen from guar gum, xanthan gum, spruce meal, cellulose, modified derivatives thereof, and synthetic polymers.

30. Composition according to Claim 26, **characterized in that** the sunscreens are chosen from anthranilates; cinnamic derivatives; dibenzoylmethane derivatives; salicylic derivatives; camphor derivatives; triazine derivatives, preferably 1,3,5-triazine derivatives; benzophenone derivatives; β, β'-diphenylacrylate derivatives; benzotriazole derivatives; benzalmalonate derivatives; benzimidazole derivatives; imidazolines; bis-benzazolyl derivatives; p-aminobenzoic acid (PABA) derivatives; methylenebis(hydroxyphenylbenzotriazole) derivatives; screening polymers and screening silicones; α-alkylstyrene-based dimers; 4,4-diarylbutadienes, and mixtures thereof.

31. Composition according to Claim 26, **characterized in that** the dye is an oxidation dye chosen from oxidation bases and/or couplers.

32. Composition according to Claim 31, **characterized in that** the oxidation bases are chosen from ortho- and para-phenylenediamines, double bases, ortho- and para-aminophenols and heterocyclic bases, and also the addition salts of these compounds with an acid.

33. Composition according to Claim 32, **characterized in that** the heterocyclic bases are chosen from pyridine derivatives, pyrimidine derivatives and pyrazole derivatives.

34. Composition according to Claim 31, **characterized in that** the couplers are chosen from meta-phenylenediamines, meta-aminophenols, meta-diphenols and heterocyclic couplers, and the addition salts of these compounds with an acid.

35. Composition according to Claim 26, **characterized in that** the dye is a direct dye.

36. Composition according to Claim 26, **characterized in that** the pigments are chosen from titanium dioxide, zirconium oxide, zinc oxide or cerium oxide, iron oxide or chromium oxide, manganese violet, ultramarine blue, chromium hydrate, ferric blue, carbon black, and lakes based on cochineal carmine or on barium, strontium, calcium or aluminium.

37. Composition according to Claim 26, **characterized in that** the nacres or nacreous agents are chosen from mica coated with titanium or with bismuth oxychloride, titanium mica with iron oxides, titanium mica with ferric blue or chromium oxide, titanium mica with an organic pigment and nacreous pigments based on bismuth oxychloride, and insoluble organic ethers or esters.

38. Composition according to Claim 26, **characterized in that** the fixing polymer(s) is (are) chosen from cationic, anionic, amphoteric and nonionic fixing polymers, and mixtures thereof.

39. Composition according to Claim 38, **characterized in that** the cationic fixing polymer(s) is (are) chosen from homopolymers or copolymers of acrylic or methacrylic esters or amides containing amine functions, cationic polysaccharides, quaternary copolymers of vinylpyrrolidone and of vinylimidazole, cationic grafted polymers and chitosans.

40. Composition according to Claim 38, **characterized in that** the anionic fixing polymers are chosen from homopolymers or copolymers of acrylic and methacrylic acid or salts thereof, crotonic acid copolymers, monounsaturated C₄-C₈ carboxylic acid or anhydride copolymers, polyacrylamides containing carboxylate groups, homopolymers or copolymers containing sulfonic groups, anionic polyurethanes and anionic grafted silicone polymers.

41. Composition according to Claim 38, **characterized in that** the amphoteric fixing polymer(s) is (are) chosen from copolymers containing acidic vinyl units and basic vinyl units, crosslinked and acylated polyamino amides, polymers containing zwitterionic units, chitosan-based polymers, modified (C₁-C₅)alkyl vinyl ether/maleic anhydride copolymers, amphoteric polyurethanes and amphoteric grafted silicone polymers.

42. Composition according to Claim 38, **characterized in that** the nonionic fixing polymer(s) is (are) chosen from polyalkyloxazolines, vinyl acetate homopolymers and copolymers, acrylic ester homopolymers and copolymers, acrylonitrile copolymers, styrene homopolymers and copolymers, polyamides, vinyllactam homopolymers other than vinylpyrrolidone homopolymers, vinyllactam copolymers, nonionic polyurethanes and nonionic grafted silicone polymers.

43. Composition according to Claim 26, **characterized in that** the conditioner is chosen from natural or synthetic oils, natural or synthetic waxes, cationic surfactants and conditioning cationic polymers.

44. Composition according to any one of Claims 26 to 43, **characterized in that** the cosmetically active compound(s) is (are) present in the composition in contents of between 0.0001% and 60%, preferably between 0.01% and 30% and more preferably between 0.1% and 15% by weight relative to the total weight of the composition.

45. Composition according to any one of Claims 26 to 44, **characterized in that** the size of the crosslinked polymer particles is between 1 and 100 nm and preferably between 1 and 50 nm.

46. Composition according to any one of Claims 26 to 45, **characterized in that** the crosslinked network of crosslinked polymer particles consists of particles with a mean spacing of from 50 to 1000 nm and preferably from 50 to 500 nm.

47. Composition according to any one of Claims 26 to 46, **characterized in that** the crosslinked polymer particles are present in an amount of between 0.4% and 60%, preferably between 1% and 30% and more preferably between 2% and 20% relative to the total weight of the composition.

48. Composition according to any one of Claims 26 to 47, **characterized in that** the cosmetically acceptable medium contains water or one or more cosmetically acceptable solvents, or a mixture of water and of one or more cosmetically acceptable solvents.

49. Composition according to any one of Claims 26 to 48, **characterized in that** the cosmetically acceptable medium contains water.

50. Cosmetic gel, **characterized in that** it comprises a composition according to any one of Claims 26 to 49.

51. Cosmetic hair process, **characterized in that** it comprises the application to the hair of a composition comprising, in a cosmetically acceptable medium, at least one cosmetically active compound and a gel comprising at least one crosslinked network of crosslinked polymer particles.

52. Process according to Claim 51, **characterized in that** it also comprises, simultaneously with or, preferably, consecutive to the application of the composition to the hair, exposure of the hair to heat.

## Patentansprüche

1. Verwendung eines Gels, das mindestens ein vernetztes Netzwerk von Partikeln eines vernetzten Polymers enthält, für die kosmetische Behandlung von Keratinfasern und insbesondere Haaren.

2. Verwendung nach Anspruch 1, **dadurch gekennzeichnet, dass** das Polymer auf einen äußeren Reiz reagiert.

3. Verwendung nach Anspruch 2, **dadurch gekennzeichnet, dass** es sich bei dem äußeren Reiz um die Temperatur oder UV-Strahlung handelt.

4. Verwendung nach einem der Ansprüche 1 bis 3, **dadurch gekennzeichnet, dass** die kosmetisch wirksame Verbindung unter den Reduktionsmitteln oder Antioxidantien, Oxidationsmitteln, Siliconen, beruhigenden Stoffen, Schaumverhütungsmitteln, Hydratisierungsmitteln, Emollientien, Weichmachern, Sonnenschutzfiltern, Farbstoffen, Pigmenten, Füllstoffen, Tonen, Perlmuttpigmenten oder Perlglanzpigmenten, Parfums, peptisierenden Stoffen, Verdickungsmitteln, Konservierungsmitteln, fixierenden Polymeren, Vitaminen oder Provitaminen, Konditioniermitteln, kationischen Polymeren, kationischen grenzflächenaktiven Stoffen, Wachsen und Proteinen ausgewählt ist.

5. Verwendung nach Anspruch 4, **dadurch gekennzeichnet, dass** das Reduktionsmittel ausgewählt ist unter NaBH₄, KBH₄, BH₃, NADPH, NADH, H₂, 9-BBN (9-Borabicyclo[3.3.1]nonan), LiAlH₄, NaAlEt₂H₂, Na₂S₂O₃, Diisoamylboran, Ascorbinsäure und cyclischen oder aliphatischen Verbindungen, die die chemische Endiol-Gruppe -(OH)C=C(OH)- enthalten, wobei die isomeren Formen, deren Salze und deren Ester eingeschlossen sind, Hydrochinon, NaBH(OAc)₃, Formamidinsulfinsäure (FASA), N-Phenylformamidinsulfinsäure, Phosphinen, NaBH₃CN, Na₂S₂O₄, BMS (Bismercaptoethylsulfon), DTT (Dithiothreitol), DMH (N,N'-Dimethyl-N,N'-bis(mercaptoacetyl)hydrazin), Thiolen, Thiolaten, Citraten, Sulfiten, Bisulfiten, Monopersulfaten und Bisulfaten von Alkalimetallen, Protein-Disulfid-Isomerase, Thioredoxin und Sulfinaten.

6. Verwendung nach Anspruch 4, **dadurch gekennzeichnet, dass** das Oxidationsmittel unter Wasserstoffperoxid, Bromaten und Salzen von Persäuren ausgewählt ist.

7. Verwendung nach Anspruch 4, **dadurch gekennzeichnet, dass** das Verdickungsmittel unter Guargummi, Xanthangummi, Fichtenmehl, Cellulose, ihren modifizierten Derivaten und synthetischen Polymeren ausgewählt ist.

8. Verwendung nach Anspruch 4, **dadurch gekennzeichnet, dass** die Sonnenschutzfilter unter Anthranilaten; Zimtsäurederivaten; Dibenzoylmethanderivaten; Salicylsäurederivaten; Campherderivaten; Triazinderivaten, vorzugsweise 1,3,5-Triazinderivaten; Benzophenonderivaten; _,_'-Diphenylacrylatderivaten; Benzotriazolderivaten; Benzalmalonatderivaten; Benzimidazolderivaten; Imidazolinen; Bis-benzoazolylderivaten; Derivate von *p*-Aminobenzoesäure (PABA); Methylen-bis-(hydroxyphenylbenzotriazolderivaten); polymeren Filtern und Siliconfiltern; von _-Alkylstyrol abgeleiteten Dimeren; 4,4-Diarylbutadienen und deren Gemischen ausgewählt sind.

9. Verwendung nach Anspruch 4, **dadurch gekennzeichnet, dass** das Farbmittel ein Oxidationsfarbstoff ist, der unter den Oxidationsbasen und/oder Kupplern ausgewählt ist.

10. Verwendung nach Anspruch 9, **dadurch gekennzeichnet, dass** die Oxidationsbasen unter *o*- und *p*-Phenylendiaminen, Doppelbasen, *o*- und *p-*Aminophenolen, heterocyclischen Basen sowie den Additionssalzen dieser Verbindungen mit einer Säure ausgewählt sind.

11. Verwendung nach Anspruch 10, **dadurch gekennzeichnet, dass** die heterocyclischen Basen unter den Pyridinderivaten, Pyrimidinderivaten und Pyrazolderivaten ausgewählt sind.

12. Verwendung nach Anspruch 9, **dadurch gekennzeichnet, dass** die Kuppler unter den *m-* Phenylendiaminen, *m-* Aminophenolen, *m-* Dihydroxybenzolen, heterocyclischen Kupplern und den Additionssalzen dieser Verbindungen mit einer Säure ausgewählt sind.

13. Verwendung nach Anspruch 4, **dadurch gekennzeichnet, dass** der Farbstoff ein Direktfarbstoff ist.

14. Verwendung nach Anspruch 4, **dadurch gekennzeichnet, dass** die Pigmente unter Titandioxid, den Oxiden von Zirconium, Zink oder Cer, den Oxiden von Eisen oder Chrom, Manganviolett, Ultramarinblau, Chromhydrat, Eisenblau, Ruß und den Lacken von Cochenillekarmin, Barium, Strontium, Calcium oder Aluminium ausgewählt sind.

15. Verwendung nach Anspruch 4, **dadurch gekennzeichnet, dass** die Perlmuttpigmente oder Perlglanzpigmente unter den mit Titan oder Bismutoxidchlorid überzogenen Glimmer-Pigmenten, Titan-Glimmer-Pigmenten mit Eisenoxiden, Titan-Glimmer-Pigmenten mit Eisenblau oder Chromoxid, Titan-Glimmer-Pigmenten mit einem organischen Pigment und den Perlglanzpigmenten auf der Basis von Bismutoxidchlorid, unlöslichen organischen Ethern oder Estern ausgewählt sind.

16. Verwendung nach Anspruch 4, **dadurch gekennzeichnet, dass** das oder die fixierende(n) Polymer(e) unter den kationischen, anionischen, amphoteren, nichtionischen fixierenden Polymeren und deren Gemischen ausgewählt sind.

17. Verwendung nach Anspruch 16, **dadurch gekennzeichnet, dass** das oder die kationische(n) fixierende(n) Polymer(e) unter den Homopolymeren oder Copolymeren von Acrylsäureestern, Methacrylsäureestern, Acrylamiden oder Methacrylamiden mit aminierten Funktionen, kationischen Polysacchariden, quartären Copolymeren von Vinylpyrrolidon und Vinylimidazol, gepfropften kationischen Polymeren und Chitosanen ausgewählt sind.

18. Verwendung nach Anspruch 16, **dadurch gekennzeichnet, dass** das oder die anionische(n) fixierende(n) Polymer(e) unter den Homopolymeren oder Copolymeren von Acrylsäure oder Methacrylsäure oder ihren Salzen, Copolymeren von Crotonsäure, Copolymeren von einfach ungesättigten Carbonsäuren oder Carbonsäureanhydriden mit 4 bis 8 Kohlenstoffatomen, Polyacrylamiden mit Carboxylatgruppen, Homopolymeren oder Copolymeren mit Sulfonsäuregruppen, anionischen Polyurethanen und anionischen gepfropften Siliconpolymeren ausgewählt sind.

19. Verwendung nach Anspruch 16, **dadurch gekennzeichnet, dass** das oder die amphotere(n) fixierende(n) Polymer(e) unter den Copolymeren mit sauren Vinyleinheiten und basischen Vinyleinheiten, vernetzten und acylierten Polyaminoamiden, Polymeren mit zwitterionischen Einheiten, von Chitosan abgeleiteten Polymeren, modifizierten Alkyl(C₁-C₅)vinylether/Maleinsäureanhydrid-Copolymeren, amphoteren Polyurethanen und amphoteren gepfropften Siliconpolymeren ausgewählt sind.

20. Verwendung nach Anspruch 16, **dadurch gekennzeichnet, dass** das oder die nichtionische(n) fixierende(n) Polymer(e) unter den Polyalkyloxazolinen, Homopolymeren und Copolymeren von Vinylacetat, Homopolymeren und Copolymeren von Acrylestern, Copolymeren von Acrylnitril, Homopolymeren und Copolymeren von Styrol, Polyamiden, Homopolymeren von Vinyllactam, die von den Homopolymeren von Vinylpyrrolidon verschieden sind, Copolymeren von Vinyllactam, nichtionischen Polyurethanen und nichtionischen gepfropften Siliconpolymeren ausgewählt sind.

21. Verwendung nach Anspruch 4, **dadurch gekennzeichnet, dass** das Konditioniermittel unter den natürlichen oder synthetischen Ölen, natürlichen oder synthetischen Wachsen, kationischen grenzflächenaktiven Stoffen und konditionierenden kationischen Polymeren ausgewählt ist.

22. Verwendung nach einem der vorhergehenden Ansprüche, **dadurch gekennzeichnet, dass** die Partikelgröße des vernetzten Polymers im Bereich von 1 bis 100 nm und vorzugsweise 1 bis 50 nm liegt.

23. Verwendung nach einem der vorhergehenden Ansprüche, **dadurch gekennzeichnet, dass** das vernetzte Netzwerk von Partikeln eines vernetzten Polymers aus Partikeln besteht, die einen mittleren Abstand von 50 bis 1.000 nm und vorzugsweise 50 bis 500 nm haben.

24. Verwendung nach einem der vorhergehenden Ansprüche für die Festigung und/oder Formgebung von Keratinfasern und insbesondere Haaren.

25. Verwendung eines Gels, das mindestens ein vernetztes Netzwerk von Partikeln eines vernetzten Polymers enthält, für die Herstellung einer haarkosmetischen Zusammensetzung.

26. Kosmetische Zusammensetzung und insbesondere haarkosmetische Zusammensetzung, die in einem kosmetisch akzeptablen Medium ein Gel, das mindestens ein vernetztes Netzwerk von Partikeln eines vernetzten Polymers umfasst, und mindestens eine kosmetisch wirksame Verbindung enthält, die unter den Reduktionsmitteln oder Antioxidantien, Oxidationsmitteln, Siliconen, beruhigenden Stoffen, Schaumverhütungsmitteln, Hydratisierungsmitteln, Emollientien, Weichmachern, Sonnenschutzfiltern, Farbmitteln, Pigmenten, Füllstoffen, Tonen, Perlmuttpigmenten oder Perlglanzpigmenten, Parfums, Verdickungsmitteln, fixierenden Polymeren, Vitaminen oder Provitaminen, Konditioniermitteln, kationischen Polymeren, kationischen grenzflächenaktiven Stoffen und Wachsen ausgewählt ist.

27. Zusammensetzung nach Anspruch 26, **dadurch gekennzeichnet, dass** das Reduktionsmittel ausgewählt ist unter NaBH₄, KBH₄, BH₃, NADPH, NADH, H₂, 9-BBN (9-Borabicyclo[3.3.1]nonan), LiAlH₄, NaAlEt₂H₂, Na₂S₂O₃, Diisoamylboran, Ascorbinsäure und cyclischen oder aliphatischen Verbindungen, die die chemische Endiol-Gruppe -(OH)C=C(OH)- enthalten, wobei die isomeren Formen, deren Salze und deren Ester eingeschlossen sind, Hydrochinon, NaBH(OAc)₃, Formamidinsulfinsäure (FASA), N-Phenylformamidinsulfinsäure, Phosphinen, NaBH₃CN, Na₂S₂O₄, BMS (Bismercaptoethylsulfon), DTT (Dithiothreitol), DMH (N,N'-Dimethyl-N,N'-bis(mercaptoacetyl)hydrazin), Thiolen, Thiolaten, Citraten, Sulfiten, Bisulfiten, Monopersulfaten und Bisulfaten von Alkalimetallen, Protein-Disulfid-Isomerase, Thioredoxin und Sulfinaten.

28. Zusammensetzung nach Anspruch 26, **dadurch gekennzeichnet, dass** das Oxidationsmittel ausgewählt ist unter Wasserstoffperoxid, Bromaten und Persalzen.

29. Zusammensetzung nach Anspruch 26, **dadurch gekennzeichnet, dass** das Verdickungsmittel ausgewählt ist unter Guargummi, Xanthangummi, Fichtenmehl, Cellulose, ihren modifizierten Derivaten, synthetischen Polymeren.

30. Zusammensetzung nach Anspruch 26, **dadurch gekennzeichnet, dass** die Sonnenschutzfilter unter Anthranilaten; Zimtsäurederivaten; Dibenzoylmethanderivaten; Salicylsäurederivaten; Campherderivaten; Triazinderivaten, vorzugsweise 1,3,5-Triazinderivaten; Benzophenonderivaten; _,_'-Diphenylacrylatderivaten; Benzotriazolderivaten; Benzalmalonatderivaten; Benzimidazolderivaten; Imidazolinen; Bis-benzoazolylderivaten; Derivaten von p-Aminobenzoesäure (PABA); Methylen-bis-(hydroxyphenylbenzotriazolderivaten); polymeren Filtern und Siliconfiltern; von _-Alkylstyrol abgeleiteten Dimeren; 4,4-Diarylbutadienen und deren Gemischen ausgewählt sind.

31. Zusammensetzung nach Anspruch 26, **dadurch gekennzeichnet, dass** das Farbmittel ein Oxidationsfarbstoff ist, der unter den Oxidationsbasen und/oder Kupplern ausgewählt ist.

32. Zusammensetzung nach Anspruch 31, **dadurch gekennzeichnet, dass** die Oxidationsbasen unter *o*- und *p*-Phenylendiaminen, Doppelbasen, *o*- und *p-*Aminophenolen, heterocyclischen Basen sowie den Additionssalzen dieser Verbindungen mit einer Säure ausgewählt sind.

33. Zusammensetzung nach Anspruch 32, **dadurch gekennzeichnet, dass** die heterocyclischen Basen unter den Pyridinderivaten, Pyrimidinderivaten und Pyrazolderivaten ausgewählt sind.

34. Zusammensetzung nach Anspruch 31, **dadurch gekennzeichnet, dass** die Kuppler unter den *m*-Phenylendiaminen, *m*-Aminophenolen, *m-*Dihydroxybenzolen, heterocyclischen Kupplern und den Additionssalzen dieser Verbindungen mit einer Säure ausgewählt sind.

35. Zusammensetzung nach Anspruch 26, **dadurch gekennzeichnet, dass** der Farbstoff ein Direktfarbstoff ist.

36. Zusammensetzung nach Anspruch 26, **dadurch gekennzeichnet, dass** die Pigmente unter Titandioxid, den Oxiden von Zirconium, Zink oder Cer, den Oxiden von Eisen oder Chrom, Manganviolett, Ultramarinblau, Chromhydrat, Eisenblau, Ruß und den Lacken von Cochenillekarmin, Barium, Strontium, Calcium oder Aluminium ausgewählt sind.

37. Zusammensetzung nach Anspruch 26, **dadurch gekennzeichnet, dass** die Perlmuttpigmente oder Perlglanzpigmente unter den mit Titan oder Bismutoxidchlorid überzogenen Glimmer-Pigmenten, Titan-Glimmer-Pigmenten mit Eisenoxiden, Titan-Glimmer-Pigmenten mit Eisenblau oder Chromoxid, Titan-Glimmer-Pigmenten mit einem organischen Pigment und den Perlglanzpigmenten auf der Basis von Bismutoxidchlorid, unlöslichen organischen Ethern oder Estern ausgewählt sind.

38. Zusammensetzung nach Anspruch 26, **dadurch gekennzeichnet, dass** das oder die fixierende(n) Polymer(e) unter den kationischen, anionischen, amphoteren, nichtionischen fixierenden Polymeren und deren Gemischen ausgewählt sind.

39. Zusammensetzung nach Anspruch 38, **dadurch gekennzeichnet, dass** das oder die kationische(n) fixierende(n) Polymer(e) unter den Homopolymeren oder Copolymeren von Acrylsäureestern, Methacrylsäureestern, Acrylamiden oder Methacrylamiden mit aminierten Funktionen, kationischen Polysacchariden, quartären Copolymeren von Vinylpyrrolidon und Vinylimidazol, gepfropften kationischen Polymeren und Chitosanen ausgewählt sind.

40. Zusammensetzung nach Anspruch 38, **dadurch gekennzeichnet, dass** das oder die anionische(n) fixierende(n) Polymer(e) unter den Homopolymeren oder Copolymeren von Acrylsäure oder Methacrylsäure oder ihren Salzen, Copolymeren von Crotonsäure, Copolymeren von einfach ungesättigten Carbonsäuren oder Carbonsäureanhydriden mit 4 bis 8 Kohlenstoffatomen, Polyacrylamiden mit Carboxylatgruppen, Homopolymeren oder Copolymeren mit Sulfonsäuregruppen, anionischen Polyurethanen und anionischen gepfropften Siliconpolymeren ausgewählt sind.

41. Zusammensetzung nach Anspruch 38, dass das oder die amphotere(n) fixierende(n) Polymer(e) unter den Copolymeren mit sauren Vinyleinheiten und basischen Vinyleinheiten, vernetzten und acylierten Polyaminoamiden, Polymeren mit zwitterionischen Einheiten, von Chitosan abgeleiteten Polymeren, modifizierten Alkyl(C₁-C₅)vinylether/Maleinsäureanhydrid-Copolymeren, amphoteren Polyurethanen und amphoteren gepfropften Siliconpolymeren ausgewählt sind.

42. Zusammensetzung nach Anspruch 38, **dadurch gekennzeichnet, dass** das oder die nichtionische(n) fixierende(n) Polymer(e) unter den Polyalkyloxazolinen, Homopolymeren und Copolymeren von Vinylacetat, Homopolymeren und Copolymeren von Acrylestern, Copolymeren von Acrylnitril, Homopolymeren und Copolymeren von Styrol, Polyamiden, Homopolymeren von Vinyllactam, die von den Homopolymeren von Vinylpyrrolidon verschieden sind, Copolymeren von Vinyllactam, nichtionischen Polyurethanen und nichtionischen gepfropften Siliconpolymeren ausgewählt sind.

43. Zusammensetzung nach Anspruch 26, **dadurch gekennzeichnet, dass** das Konditioniermittel unter den natürlichen oder synthetischen Ölen, natürlichen oder synthetischen Wachsen, kationischen grenzflächenaktiven Stoffen und konditionierenden kationischen Polymeren ausgewählt ist.

44. Zusammensetzung nach den Ansprüchen 26 bis 43, **dadurch gekennzeichnet, dass** die kosmetisch wirksame(n) Verbindung(en) in der Zusammensetzung in Mengenanteilen von 0,0001 bis 60 %, vorzugsweise 0,01 bis 30 % und noch bevorzugter 0,1 bis 15 Gew.-% des Gesamtgewichtes der Zusammensetzung enthalten sind.

45. Zusammensetzung nach einem der Ansprüche 26 bis 44, **dadurch gekennzeichnet, dass** die Partikelgröße des vernetzten Polymers im Bereich von 1 bis 100 nm und vorzugsweise 1 bis 50 nm liegt.

46. Zusammensetzung nach einem der Ansprüche 26 bis 45, **dadurch gekennzeichnet, dass** das vernetzte Netzwerk der Partikel eines vernetzten Polymers aus Partikeln besteht, die einen mittleren Abstand von 50 bis 1.000 nm und vorzugsweise 50 bis 500 nm aufweisen.

47. Zusammensetzung nach einem der Ansprüche 26 bis 46, **dadurch gekennzeichnet, dass** die Partikel des vernetzten Polymers in einer Menge von 0,4 bis 60 %, vorzugsweise 1 bis 30 % und noch bevorzugter 2 bis 20 % des Gesamtgewichts der Zusammensetzung enthalten sind.

48. Zusammensetzung nach einem der Ansprüche 26 bis 47, **dadurch gekennzeichnet, dass** das kosmetisch akzeptable Medium Wasser oder ein oder mehrere kosmetisch akzeptable Lösungsmittel oder ein Gemisch von Wasser und einem oder mehreren kosmetisch akzeptablen Lösungsmitteln enthält.

49. Zusammensetzung nach einem der Ansprüche 26 bis 48, **dadurch gekennzeichnet, dass** das kosmetisch akzeptable Medium Wasser enthält.

50. Kosmetisches Gel, **dadurch gekennzeichnet, dass** es eine Zusammensetzung nach einem der Ansprüche 26 bis 49 enthält.

51. Kosmetisches Verfahren für die Haarbehandlung, **dadurch gekennzeichnet, dass** es das Auftragen einer Zusammensetzung, die in einem kosmetisch akzeptablen Medium mindestens eine kosmetisch wirksame Verbindung und ein Gel, das mindestens ein vernetztes Netzwerk von Partikeln eines vernetzten Polymers enthält, auf die Haare umfasst.

52. Verfahren nach Anspruch 51, **dadurch gekennzeichnet, dass** die Haare gleichzeitig mit oder vorzugsweise nach dem Aufbringen der Zusammensetzung auf die Haare Wärme ausgesetzt werden.
